(19) Europäisches Patentamt
European Patent Office
Office européen des brevets

(11) EP 3 381 905 A1

(12) EUROPEAN PATENT APPLICATION

(43) Date of publication:
03.10.2018 Bulletin 2018/40

(21) Application number: 17305371.1

(22) Date of filing: 30.03.2017

(51) Int Cl.:
*C07D 233/56* (2006.01) *C07D 233/60* (2006.01)
*C07D 405/04* (2006.01) *A61K 31/4164* (2006.01)
*A61K 31/4178* (2006.01) *A61P 31/12* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Institut National de la Santé et de la Recherche Médicale (INSERM)
75013 Paris (FR)**
- **Université de Bordeaux
33000 Bordeaux (FR)**
- **Université de Rennes 1
35000 Rennes (FR)**
- **Centre National de la Recherche Scientifique
75794 Paris Cedex 16 (FR)**

(72) Inventor: **The designation of the inventor has not yet been filed**

(74) Representative: **Nony
11 rue Saint-Georges
75009 Paris (FR)**

# (54) NOVEL ANTIVIRAL COMPOUNDS

(57) The present invention relates to a compound of formula (I):

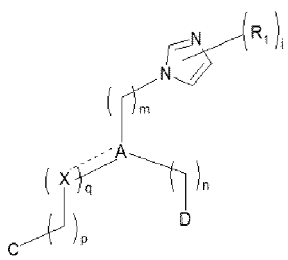

for use in the prevention and/or treatment of viral infections, and in particular by a non-enveloped DNA virus and/or an enveloped RNA virus. The present invention also relates to compounds of formula (II):

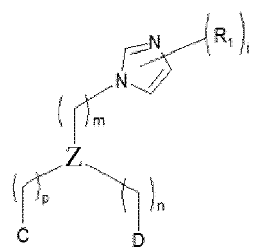

and to compositions comprising it. The inventors found that imidazole derivatives of formula (I) and (II), as defined herein, represent a novel class of antiviral agents with broad applicability against a wide spectrum of viruses.

EP 3 381 905 A1

SEQUENCE LISTING

<110> Institut National de la Sante et de la Recherche Medicale (INSERM)
Universite de Bordeaux
Centre National de la Recherche Scientifique (CNRS)
Universite de Rennes 1

<120> NOVEL ANTIVIRAL COMPOUNDS

<130> PR77223

<160> 8

<170> BiSSAP 1.3

<210> 1
<211> 20
<212> DNA
<213> Artificial Sequence

<220>
<223> GAPDH fwd primer

<400> 1
tggtatcgtg gaaggactca 20

<210> 2
<211> 20
<212> DNA
<213> Artificial Sequence

<220>
<223> GAPDH rev primer

<400> 2
ccagtagagg cagggatgat 20

<210> 3
<211> 20
<212> DNA
<213> Artificial Sequence

<220>
<223> E1B-55k fwd primer

<400> 3
gagggtaact ccagggtgcg 20

<210> 4
<211> 24
<212> DNA
<213> Artificial Sequence

<220>
<223> E1B-55k rev primer

<400> 4
tttcactagc atgaagcaac caca 24

<210> 5
<211> 24
<212> DNA
<213> Artificial Sequence

<220>
<223> E4orf6/7 fwd primer

<400> 5
acagaaccct agtattcaac ctgc 24

<210> 6
<211> 24
<212> DNA
<213> Artificial Sequence

<220>
<223> E4orf6/7 rev primer

<400> 6
gacagcgaca tgaacttaag tgag 24

<210> 7
<211> 27
<212> DNA
<213> Artificial Sequence

<220>
<223> E1A fwd primer

<400> 7
ggctcaggtt cagacacagg actgtag 27

<210> 8
<211> 21
<212> DNA
<213> Artificial Sequence

<220>
<223> E1A rev primer

<400> 8
tccggagccg cctcaccttt c 21

## Description

### FIELD OF THE INVENTION

The present application relates to antiviral compounds and to their use for the treatment and/or prevention of viral infections.

More particularly, the present invention relates to Sulconazole and Sulconazole derivatives for their use in treating and/or preventing viral infections.

### BACKGROUND OF THE INVENTION

Viruses are the causative agent in a wide variety of human infectious diseases.

Efforts to treat or prevent viral infection are generally classified into two broad categories: vaccines and antiviral drugs.

Antiviral medications are advantageously able to treat individuals who are already infected as well as preventing viral diseases where vaccination methods are seen as unavailable or unlikely.

Illustratively WO 03/063869 identifies a set of compounds altering the permeability of ion channels, which are effective in controlling the replication and/or spread of viruses belonging the Picornaviridae family (nonenveloped RNA viruses).

Accordingly, there remains a constant need to widen the therapeutic arsenal to treat and/or prevent viral diseases. This is more particularly important in view of the increasing resistance of many viruses to already known antiviral drugs.

Moreover, despite noted success in the design and development of novel antiviral molecules in recent years, existing therapies are limited in terms of the number and breadth of viruses that they may be used to treat.

Indeed, one of the main problems associated with an antiviral agent having a wide spectrum of action is that it is often toxic for the individual to which these molecules are administered.

There is thus a need for novel antiviral compounds able to prevent and/or treat viral infections. Thus, there is also a need for antiviral compounds able to prevent and/or treat a broad set of viral infections, and in particular viral infections by nonenveloped DNA viruses and enveloped RNA viruses.

There is also a need for antiviral compounds able to inhibit the propagation of a virus, to inhibit the egress of a virus from the infected cells, to inhibit the replication of a virus in cells infected by said virus and/or to reduce the nuclear accumulation of viral genomes in cells infected by said virus.

There is furthermore a need for antiviral compounds capable of targeting a variety of virus types without being toxic for the individual to which these molecules are administered.

There is also a need for antiviral compounds having a better antiviral activity than antiviral treatments already available in clinics.

### SUMMARY OF THE INVENTION

The present invention aims to meet the here-above indicated needs.

According to the inventors' experimental results, Sulconazole and its derivatives, defined as indicated hereafter, are surprisingly able to prevent and/or treat viral infections. In particular, these compounds are able to act against a wide spectrum of viruses, and in particular against viruses as different as non-enveloped DNA viruses, such as adenoviruses, and enveloped RNA viruses, such as filoviruses.

Accordingly, one of the objects of the present invention relates to a compound of formula (I):

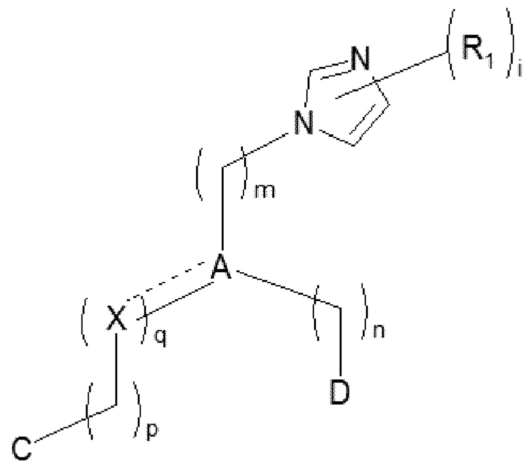

Formula (I)

wherein:

---- represents a single bond or a double bond;
q is 0 or 1;
A represents:

- CH when (i) ---- represents a single bond with q being 1 or (ii) when q is 0;
- a carbon atom when ---- represents a double bond and q is 1; or
- a heteroaromatic ring or an aromatic ring, when (i) ---- represents a single bond with q being 1, or when (ii) q is 0 ;

X, being present when q is 1, represents:

-S-;
-O-;

a -O-NH- group with ---- representing a single bond, A being linked either to the nitrogen atom or to the oxygen atom of the -O-NH- group; or
a -O-N- group with ---- representing a double bond, A being linked to the nitrogen atom of the -O-N- group;
C and D, independently, represent a non-substituted aromatic ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p, independently, are 0, 1 or 2;
i is 1, 2 or 3; and
$R_1$ independently represents a hydrogen atom, a halogen atom or a $C_1$-$C_4$ alkyl group;

or one of its pharmaceutically acceptable salts;
for use in the prevention and/or treatment of viral infections,
said compound of formula (I) being in all the possible racemic, enantiomeric and diastereoisomeric isomer forms.

In a particular embodiment, A represents a heteroaromatic ring or an aromatic ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl.

A is preferably selected from a furyl group and a phenyl group.

In a particular embodiment, X represents:

-S-; or

a -O-N- group with ---- representing a double bond, A being linked to the nitrogen atom of the -O-N- group.

In a particular embodiment, C and D, independently, represent a phenyl ring, in particular a phenyl ring substituted with one or two halogen atom, preferably one or two chlorine atom(s).

In a particular embodiment, m is 1, n is 0, p is 1 and q is 1.
In another embodiment, m is 0 or 1 and n, p and q are 0.
In a preferred embodiment, the compound of formula (I) for use according to the present invention is selected from the group consisting of:

- Sulconazole or one of its pharmaceutically acceptable salts;
- Oxiconazole or one of its pharmaceutically acceptable salts; and
- a compound of formula (II):

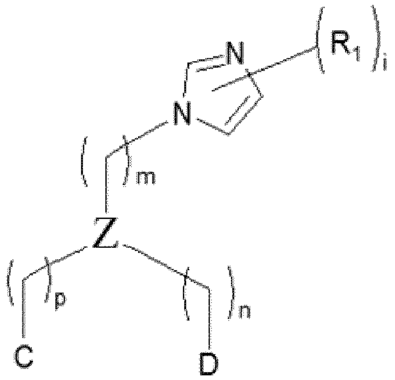

Formula (II)

wherein C, D, m, n, p, i and $R_1$ are as defined here-above; and
Z represents a saturated or unsaturated 5- or 6-membered ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl;

or one of its pharmaceutically acceptable salts;
said compound of formula (II) being in all the possible racemic, enantiomeric and diastereoisomeric isomer forms.

In a preferred embodiment, the compound of formula (I) for use according to the present invention is selected from the group consisting of:

- Sulconazole or one of its pharmaceutically acceptable salts;
- Oxiconazole or one of its pharmaceutically acceptable salts; and
- a compound of formula (I) wherein:
  - C and D are both a phenyl ring substituted with two chlorine groups;
  - m is 0 or 1;
  - i is 1;
  - n, p and q are 0;
  - $R_1$ represents an hydrogen atom; and
  - A represents a furyl or a phenyl group;

or one of its pharmaceutically acceptable salts,
said compound being in all the possible racemic, enantiomeric and diastereoisomeric isomer forms.
In particular, a compound for use according to the invention can be comprised in a composition comprising a pharmaceutically acceptable carrier, preferably in an intravenous composition.
In a particular embodiment, a compound for use according to the invention is used in the prevention and/or treatment of viral infections by a non-enveloped DNA virus and/or an enveloped RNA virus, in particular by an adenovirus and/or a filovirus.
According to a preferred embodiment, a compound for use according to the invention is used in a pharmaceutically acceptable carrier of a composition.
According to another of its objects, the present invention relates to compounds of formula (II):

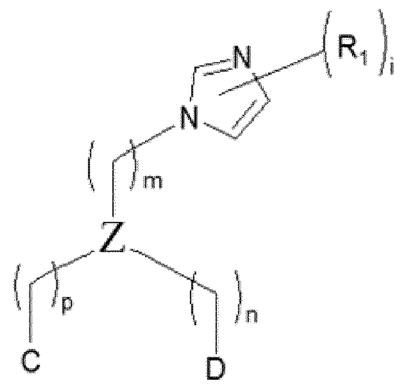

Formula (II)

wherein:

C and D, independently, represent a non-substituted ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p independently are 0, 1 or 2;
i is 1, 2 or 3;
$R_1$ represents, independently, a hydrogen atom, a halogen atom or a $C_1$-$C_4$ alkyl group, preferably a hydrogen atom; and
Z represents a saturated or unsaturated 5- or 6-membered ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl group;

or one of its pharmaceutically acceptable salts,
said compound of formula (II) being in all the possible racemic, enantiomeric and diastereoisomeric isomer forms.

In a particular embodiment, C, D, m, n and p are as defined here-above.

In a preferred embodiment, a compound of formula (II) of the invention is selected from the group consisting of:

(i) a compound wherein:

- C and D both represent a phenyl ring substituted with two chlorine groups;
- m, n and p are 0;
- Z represents a furyl group; and
- $R_1$ represents an hydrogen atom and i is 1;

and
(ii) a compound wherein:

- C and D both represent a phenyl ring substituted with two chlorine groups;
- m is 1;
- n and p are 0;
- Z is a phenyl group; and
- $R_1$ represents an hydrogen atom and i is 1.

A compound according to the invention notably possesses the following advantageous properties:

(i) they possess an antiviral activity, in particular against very different viruses such as non-enveloped DNA viruses, for example adenoviruses, and enveloped RNA viruses, for example filoviruses;
(ii) they are able to inhibit the propagation of a virus;
(iii) they are able to inhibit the replication of a virus in infected cells;
(iv) they are able to reduce the nuclear accumulation of viral genomes in cells infected by said virus; and

(v) they are not toxic.

The compounds of the present invention moreover provide the advantage of being more efficient that a standard clinical antiviral drug: Cidofovir™, as demonstrated in the following examples.
According to one embodiment, the present invention thus relates to a compound of formula (I):

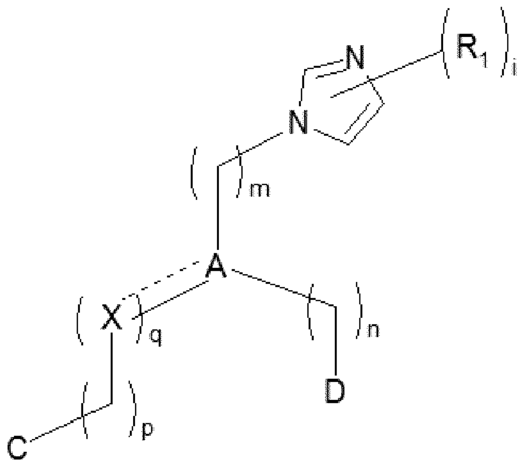

Formula (I)

wherein:

---- represents a single bond or a double bond;
q is 0 or 1;
A represents:

- CH when (i) ---- represents a single bond with q being 1 or (ii) when q is 0;
- a carbon atom when ---- represents a double bond and q is 1; or
- a heteroaromatic ring or an aromatic ring, when (i) ---- represents a single bond with q being 1, or when (ii) q is 0 ;

X, being present when q is 1, represents:

- S-;
- O-;

a -O-NH- group with ---- representing a single bond, A being linked either to the nitrogen atom or to the oxygen atom of the -O-NH- group; or
a -O-N- group with ---- representing a double bond, A being linked to the nitrogen atom of the -O-N- group;
C and D, independently, represent a non-substituted aromatic ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p, independently, are 0, 1 or 2;
i is 1, 2 or 3; and
$R_1$ independently represents a hydrogen atom, a halogen atom or a $C_1$-$C_4$ alkyl group;

or one of its pharmaceutically acceptable salts;
for use in the prevention and/or treatment of viral infections by non-enveloped DNA viruses and enveloped RNA viruses, said compound of formula (I) being in all the possible racemic, enantiomeric and diastereoisomeric isomer forms.
According to one embodiment, the compound of formula (I), is as previously defined, with the proviso that said compound of formula (I) is not of formula:

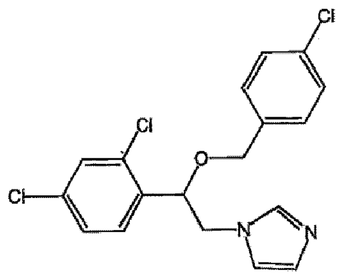
.

Another object of the present invention relates to a composition comprising, in a pharmaceutically acceptable carrier, at least one compound of formula (II) according to the invention.

## FIGURE LEGENDS

**Figure 1**: represents the effect of DMSO or Sulconazole (5μM) on adenovirus spreading in HEK293 cells infected with recombinant E1-deficient GFP expressing HAd-C5-GFP at 0.1 physical particle per cell for 24 hours followed by inoculums removal and overlay with agarose supplemented with 5μM of DMSO (negative control) or Sulconazole. Plaque sizes were measured after 6 days post infection. The mean size was determined from about 30 plaques derived from three independent experiments. Abscissa: compound administered. Ordinate: Plaque size (pixel). (****P<0.0001)

**Figure 2(A)**: represents the effect of Sulconazole on Marburg VP40 egress. HEK293T cells were transfected with wt MARV VP40 ($VP40_{WT}$) or an empty plasmid (mock). Transfected cells received 5μM of Sulconazole, 1μM of N4 or DMSO as negative control for 24 hours. VLPs (virus-like particles) (top) and cell lysates (bottom) were analyzed by western blot and VPL release was quantified (graph below western blot images). Error bars represent the SD from three independent experiments. Abscissa: compound administered. Ordinate: % of VP40 VLP release (**P<0.01)

**Figure 2(B):** represents the effect of Sulconazole on Marburg VP40 egress with Ebola *late domain.* HEK293T cells were transfected and analyzed as in Figure 2(A) using MARV VP40 with the *late domain* motif of Ebola virus (PTAPPEY) VP40 ($VP40_{ELD}$), or an empty plasmid (mock). VLPs (virus-like particles) (top) and cell lysates (bottom) were analyzed by western blot and VPL release was quantified (graph below western blot images). Error bars represent the SD from three independent experiments. Abscissa: compound administered. Ordinate: % of VP40 VLP release

(**P<0.01) (***P<0.005)

**Figure 3(A)**: represents the effect of Sulconazole on infectious Marburg virus replication. Cells are infected with infectious MARV in presence of 5 μM or 10 μM of Sulconazole or DMSO as negative control. 24 hours post infection, viral titers (TCID50) are determined by endpoint titration. Viral titers are show in presence Sulconazole (grey bar) or DMSO (black bar) when infections are carried out in presence of the Sulconazole or DMSO.

Abscissa: compound administered (DMSO or Sulconazole) and quantity administered (5 μM or 10 μM). Ordinate: Virus titer ($TCID_{50}$/mL).

**Figure 3 (B):** represents the effect of Sulconazole on infectious Marburg virus replication. Cells are infected with infectious MARV in presence of 5 μM or 10 μM of Sulconazole or DMSO as negative control. 24 hours post infection, viral titers (TCID50) are determined by endpoint titration. Viral titers are show in presence Sulconazole (grey bar) or DMSO (black bar) when Sulconazole or DMSO are added 5 hours post infection.

Abscissa: compound administered (DMSO or Sulconazole) and quantity administered (5 μM or 10 μM). Ordinate: Virus titer ($TCID_{50}$/mL).

**Figure 4:** represents the effect of Sulconazole on early gene expression. U2OS cells are infected for 4 hours with replicative HAd-C5 or an empty plasmid in presence of 5 μM of Sulconazole or DMSO as negative control. Adenoviral early gene expression is quantified by real time PCR using primers for E1A, E1B or E4 orf6/7 cDNA and normalized by the $2^{-\Delta\Delta Ct}$ method. Error bars represent the SD variation of the values from triplicate reactions of two experiments. Abscissa: compound administered (DMSO or Sulconazole) or negative control with an empty vector. Ordinate: arbitrary unit.

(*P<0.05) (***P<0.005)

**Figure 5(A)**: represents the effect of Sulconazole on adenovirus genome nuclear import. U2OS cells were infected as in Figure 4 for 2 hours, fixed and stained with antibodies against protein VII in presence of 5 μM Sulconazole or

DMSO negative control. Nuclear protein VII dots were quantified using ImageJ and given as box-plots in absolute numbers per nucleus (n= >100 cells). (****P<0.0001)
Abscissa: compound administered (DMSO or Sulconazole). Ordinate: pVII punctae per nuclei.
**Figure 5(B):** represents the representative field of view at 2 hours post infection. On the left are represented cells treated with DMSO as indicated in Figure 5(A). On the right are represented cells treated with Sulconazole.
**Figure 6:** Effect of Sulconazole and its derivatives on adenovirus spread. Plaque assays are performed as described in Figure 1 and treated with Sulconazole, Oxiconazole, CT2-10 or CT2-13 at 5 μM. Cidofovir™ was also tested at 10 μM or 20 μM. Plaque sizes were determined and are shown as box-plots (n>74).
Abscissa: compound administered. Ordinate: Plaque size (pixel). (**P<0.01) (****P<0.0001)
**Figure 7:** Toxicity measure of Sulconazole, CT2-10, CT2-13 and Oxiconazole on U2OS cells.
Abscissa: Tested compound concentration (μm). Ordinate: Normalized cell viability (%).

## DETAILED DESCRIPTION OF THE INVENTION

### Definitions

As used herein:

- "prevention" or "prevent" means at least partly reducing the risk of manifestation of a given phenomenon, i.e., in the present invention, a viral infection of an individual. A partial reduction implies that the risk remains, but to a lesser extent than before implementing the invention. Thus, "prevention" may also encompass the reduction of likelihood of manifestation of a given phenomenon, i.e., in the present invention, a viral infection of an individual.
- "inhibition" or "inhibit" means totally or partially reducing the manifestation of a given phenomenon, i.e., in the present invention, a viral infection, the propagation of a viral infection, the replication of a virus in infected cells and/or the nuclear accumulation of viral genomes in cells infected with a virus. Accordingly, inhibition may also refer to a reduction in viral load and/or pathogenicity.
- "pharmaceutically acceptable carrier" refers to a carrier medium which does not interfere with the effectiveness of the biological activity of the active ingredient(s) and which is not excessively toxic to the host at the concentration at which it is administered. The use of such media for pharmaceutically active substances is well known in the art (see for example "Remington's Pharmaceutical Sciences", E. W. Martin, 18th Ed., 1990, Mack Publishing Co.: Easton, Pa.).
- "individual" is intended for an animal, including human beings, affected or likely to be affected with a virus infection according to the invention. Said animal can in particular be intended for livestock, such as cattle, pigs and poultry; other non-human mammals such as pet, zoo or sports animals ; or human beings, affected or likely to be affected with a virus infection according to the invention. Said individual is preferably a human being.
- "viral infection" and "infected with a virus" as used herein mean that said human or non-human has been exposed to a pathogenic virus RNA or DNA and that said virus is attached to one or more cells of the host then penetrated (or may enter) into said one or more cells and have (or may have) detrimental effects for at least one cell of said animal or human.

In particular such viral infection is able to evolve towards clinical signs or accompanying said infection induced pathologies. Thus, a "viral infection" within the meaning of the present invention includes both the earliest phases of viral contamination, as well as the later phases and intermediate phases of viral contamination.

- pharmaceutically acceptable salts includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.
Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.
The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of

preparation or crystallization, or adventitious to such solvent. Such solvates are within the scope of the present invention.

In the context of the present, invention the terms below have the following meanings:

- a halogen atom: a fluorine, a chlorine, a bromine or an iodine;
- Ct-Cz: a carbon chain that can have from t to z carbon atoms, where t and z may have the values from 1 to 7; for example, $C_1$-$C_4$ is a carbon chain that may have from 1 to 4 carbon atoms;
- $C_1$-$C_4$ alkyl as used herein respectively refers to $C_1$-$C_4$ normal, secondary or tertiary saturated hydrocarbon. Non limiting examples are methyl, ethyl, propyl, isopropyl, butyl, isobutyl or tertbutyl;
- $C_1$-$C_4$ alkoxy is intended to mean an -O-($C_1$-$C_4$)alkyl radical where the $C_1$-$C_4$ alkyl group is as defined above. Non limiting examples are methoxy, ethoxy, propoxy, isopropoxy, butoxy, sec-butoxy or tert-butoxy;
- an aromatic ring refers to a mono or polycyclic, preferably a monocyclic, aromatic hydrocarbon radical of 6-20 atoms, preferably 6 atoms, derived by the removal of one hydrogen from a carbon atom of a parent aromatic ring system. An aromatic ring of the invention is preferably a phenyl group;
- an heteroaromatic ring denotes a 5- or 6-membered aromatic ring comprising 1 or 2 heteroatoms;
- a heteroatom is understood to mean nitrogen, oxygen or sulphur;
- when q is 0 in formula (I) of the present invention, it means that X and ---- are not present.

The present inventors have performed a huge amount of work with the view of identifying novel substances endowed with antiviral properties. In particular, the inventors managed to unexpectedly identify compounds of formula (I) according to the invention having a wide antiviral scope of action and not toxic for the individual to which they are administered. Even more surprisingly, the compounds of the present invention have a better antiviral activity than a standard clinical antiviral drug: Cidofovir™.

More particularly, new compounds having the formula (II) according to the invention have been synthesized by the inventors as described here-after.

**Sulconazole and derivatives thereof**

Sulconazole is a well-known antifungal agent of the imidazole class mainly used to treat skin infections such as athlete's foot, jock itch, and ringworm. However, to the knowledge of the inventors, the antiviral properties of this compound have never before been described.

Sulconazole has the following formula:

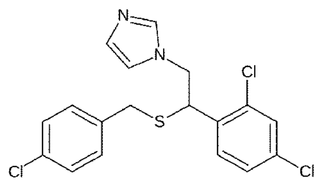

The inventors have unexpectedly found that this compound, as well as its derivatives defined here-after, possess an antiviral activity, and more importantly an antiviral activity against a wide spectrum of viruses, including both non-enveloped DNA viruses, such as adenoviruses, and enveloped RNA viruses, such as filoviruses.

This is surprising because Sulconazole was not previously known to possess anti-viral properties, and its antifungal activity was itself with ill-defined mode-of-action.

Without wishing to be bound by the theory, the inventors are of the opinion that Sulconazole and derivatives thereof behave as antiviral agents by perturbing the interaction between NEDD4 ubiquitin ligases and viral [LP]PxY motifs.

NEDD4 ubiquitin ligases of the HECT-E3 family are evolutionary conserved in Eukaryotes. Also, several virus families contain [LP]PxY peptide motifs embedded in their genome-encoded proteins. Viral [LP]PxY motifs belong to a group of short viral peptide motifs historically named *late domains* due to their prominent role at late stages in the lifecycle of several enveloped viruses.

Accordingly, the inventors are of the opinion that Sulconazole derivatives represent a novel class of antiviral agents with broad applicability.

Accordingly, the present invention relates to a compound of formula (I) as defined here-after for its use in the prevention and/or treatment of viral infections.

The compounds of formula (I) according to the invention are as follows:

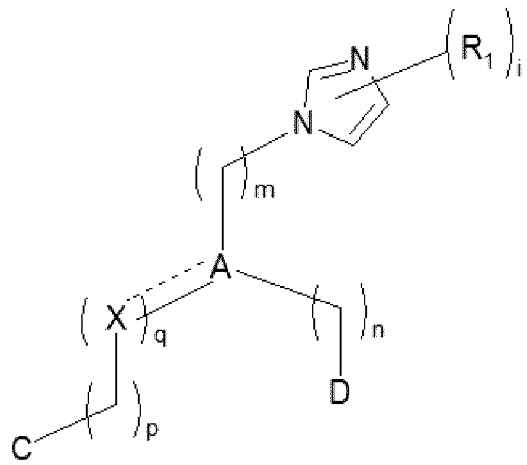

Formula (I)

wherein:

---- represents a single or a double bond;
q is 0 or 1;
A represents:

- CH when (i) ---- represents a single bond with q being 1 or (ii) when q is 0;
- a carbon atom when ---- represents a double bond and q is 1; or
- a heteroaromatic ring or an aromatic ring, when (i) ---- represents a single bond with q being 1, or when (ii) q is 0 ;

X, being present when q is 1, represents:

- S-;
- O-;

a -O-NH- group with ---- representing a single bond, A being linked either to the nitrogen atom or to the oxygen atom of the -O-NH- group; or
a -O-N- group with ---- representing a double bond, A being linked to the nitrogen atom of the -O-N- group; C and D, independently, represent a non-substituted aromatic ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p, independently, are 0, 1 or 2;
i is 1, 2 or 3; and
$R_1$ independently represents a hydrogen atom, a halogen atom or a $C_1$-$C_4$ alkyl group;

or one of its pharmaceutically acceptable salts.

In a particular embodiment, A is selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl.

In a particular embodiment, A is an aromatic ring, and is preferably a phenyl group.

In another embodiment, A is an heteroaromatic ring, and is preferably a furyl group.

In a preferred embodiment, A is selected from a furyl group or a phenyl group. According to a preferred embodiment, A represents:

- CH;
- a carbon atom when ---- represents a double bond and q is 1;
- a furyl group; or
- a phenyl group.

In a particular embodiment, X, when present, i.e. when q is 1, represents:

- S-;
- a -O-NH- group with ---- representing a single bond, A being linked either to the nitrogen atom or to oxygen atom of the -O-NH- group; or
- a -O-N- group with ---- representing a double bond, A being linked to the nitrogen atom of the -O-N- group.

In a preferred embodiment, X, when q is 1, represents:

- S-; or
- a -O-N- group with ---- representing a double bond, A being linked to the nitrogen atom of the -O-N- group.

In a particular embodiment, C and D, independently, represent a phenyl ring, preferably a substituted phenyl ring, in particular a phenyl ring substituted with one or two halogen atom, preferably one or two chlorine atom(s).
According to a particular embodiment, C and D, independently, represent a phenyl ring substituted with at least one halogen atom, preferably 1 or 2 halogen atoms, more preferably 1 or 2 chlorine atom(s).
C and D can in particular be selected, independently, from phenyl rings substituted with at least one halogen atom, in particular from phenyl rings substituted with 1 or 2 halogen atoms, more preferably 1 or 2 chlorine atom(s).
In a particular embodiment, C and D are identical. According to this embodiment, C and D can both preferably represent a phenyl ring substituted with two chlorine atoms.
In another embodiment, C and D are different. According to this embodiment, C or D can represent a phenyl ring substituted with two chlorine atoms while the other one from C and D represent a phenyl ring substituted with one chlorine atom. According to this embodiment, D represents preferably a phenyl ring substituted with two chlorine atoms and C represents preferably a phenyl ring substituted with one chlorine atom.
According to a preferred embodiment, C and D are such that:

- C and D both represent a phenyl ring substituted with two chlorine atoms; or
- D represents a phenyl ring substituted with two chlorine atoms and C represents a phenyl ring substituted with one chlorine atom.

m is preferably 1.
n is preferably 0.
p is preferably 1 or 0.

In a particular embodiment, m is 1, n is 0 and/or p is 0 or 1, more particularly m is 1, n is 0 and p is 0 or 1.
Preferably m, n and p are as follows:

- m and p are 1, and n is 0; or
- m is 1, and n and p are 0.

$R_1$, independently, is preferably selected from the group consisting of a hydrogen atom and a $C_1$-$C_4$ alkyl group, and is in particular a hydrogen atom.

In an embodiment, i is 1.
In another embodiment, i is 2.
In another embodiment, i is 3.
According to one preferred embodiment, $R_1$ represents an hydrogen atom and i is 1
In a preferred embodiment, a compound of formula (I) according to the present invention is selected from the group consisting of:

- Sulconazole or one of its pharmaceutically acceptable salts:

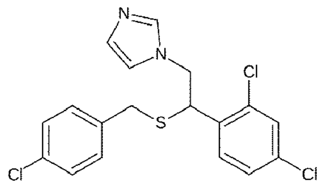;

- Oxiconazole or one of its pharmaceutically acceptable salts:

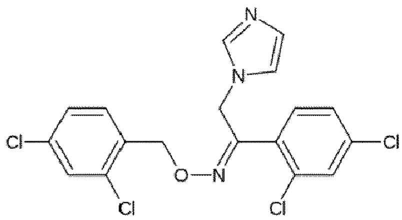;

and

- a compound of formula (II):

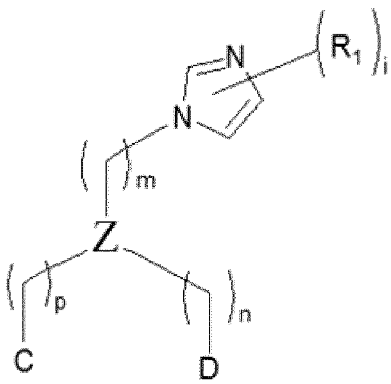

Formula (II)

wherein C, D, m, n, p, i and $R_1$ are as defined previously; and
Z represents a saturated or unsaturated 5- or 6-membered ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl;

or one of its pharmaceutically acceptable salts, said compound of formula (II) being in all the possible racemic, enantiomeric and diastereoisomeric isomer forms.

Oxiconazole is also known in the art as being an antifungal agent but, to the knowledge of the inventors, the antiviral properties of this compound have never before been described.

In particular, a compound of formula (II) described here-above is preferably such that Z represents a phenyl or a furyl group.

In a particular embodiment, C and D of a compound of formula (II), independently, represent substituted or non-substituted phenyl rings, preferably substituted phenyl rings.

According to a particular embodiment, C and D of a compound of formula (II), independently, represent substituted with at least one halogen atom, preferably 1 or 2 halogen atoms, more preferably 1 or 2 chlorine atom(s).

C and D of a compound of formula (II) can in particular represent, independently, a phenyl ring substituted with at least one halogen atom, in particular a phenyl ring substituted with 1 or 2 halogen atoms, more preferably 1 or 2 chlorine atom(s).

In a preferred embodiment, C and D of a compound of formula (II) are identical. According to this embodiment,

C and D of a compound of formula (II) can both preferably represent a phenyl ring substituted with two chlorine atoms.

m of a compound of formula (II) is preferably 1.

n of a compound of formula (II)is preferably 0.

p of a compound of formula (II) is preferably 1 or 0, and is more preferably 0.

In a preferred embodiment, a compound of formula (II) is such that m is 1 or 0, n is 0 and p is 0.

$R_1$ of a compound of formula (II) is preferably selected from the group consisting of a hydrogen atom or a $C_1$-$C_4$ alkyl group, and is in particular a hydrogen atom.

In a particular embodiment, a compound of formula (II) is selected from the group consisting of compounds wherein:

- C and D both represent a phenyl ring substituted with two chlorine groups;
- m is 0 or 1;
- n, p and i are 0; and
- Z represents a furyl or a phenyl group.

According to a particularly preferred embodiment, a compound of formula (II) of the invention is selected from the group consisting of:

- CT2-10:

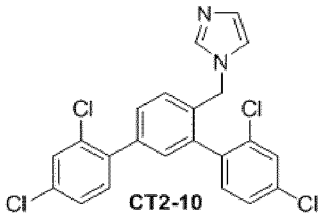

CT2-10

;

and

- CT2-13:

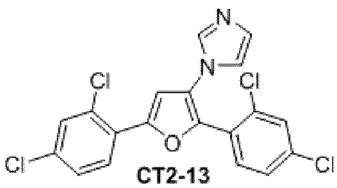

CT2-13

.

In a preferred embodiment, a compound of formula (I) according to the present invention is selected from the group consisting of:

- Sulconazole or one of its pharmaceutically acceptable salts;
- Oxiconazole or one of its pharmaceutically acceptable salts; and
- a compound of formula (I) wherein:
- C and D both represent a phenyl ring substituted with two chlorine groups;
- m and i are 1;
- n, p and q are 0; and
- A represents a furyl or a phenyl group.

A compound of formula (I) can preferably be selected from the group consisting of:

- Sulconazole or one of its pharmaceutically acceptable salts;
- Oxiconazole or one of its pharmaceutically acceptable salts;
- CT2-10:

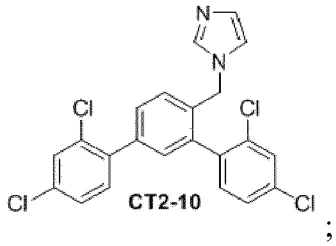

CT2-10

;

and

- CT2-13:

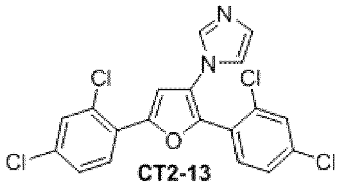

CT2-13

.

According to one embodiment, the compound of formula (I), is as previously defined, with the proviso that said compound of formula (I) is not of formula:

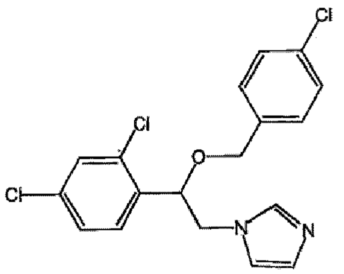

.

## Preparation of the compounds of formulae (I) and (II) of the invention

The compounds of formula (I) of the present invention may be prepared in a number of methods well known to those skilled in the art, including, but not limited to those described below, or through modifications of these methods by applying standard techniques known to those skilled in the art of organic synthesis.

They notably can be prepared according to the various methods disclosed in K.L. Kimmel et al. (J. Am. Chem. Soc. 2009, 131, 8754), in G. Bartoli et al. (Tetrahedron Lett. 1994, 35, 8651) and/or in M. Jean et al. (Org. Biomol. Chem., 2015, 13, 9168-9175).

The compounds of formula (II) of the present invention may be prepared on the basis of these methods as they are included in the general formula (I).

Furthermore, the compounds of formula (II) of the present invention may be obtained on the basis of the methods disclosed in Matsuoka et al. (Tetrahedron 62 (2006) 8199-8206) and in Altman et al. (J. Org. Chem. 2007, 72, 6190-6199).

## Application

Compounds of formulae (I) and (II) according to the invention are for use in the prevention and/or treatment of viral infections.

Viruses more particularly considered in the present invention are non-enveloped DNA viruses and/or enveloped RNA viruses.

Non-enveloped DNA viruses can in particular be selected from the group consisting of Papillomaviridae viruses, Polyomaviridae viruses, Adenoviridae viruses, Parvoviridae viruses, Reoviridae viruses, Hepevirus Norovirus, and Enterovirus.

Papillomaviridae viruses can in particular be selected from the group consisting of Alphapapillomavirus, Betapapillomavirus, Deltapapillomavirus, Dyodeltapapillomavirus, Dyoepsilonpapillomavirus, Dyoetapapillomavirus, Dyoiotapapillomavirus, Dyokappapapillomavirus, Dyolambdapapillomavirus, Dyomupapillomavirus, Dyonupapillomavirus, Dyoomikronpapillomavirus, Dyopipapillomavirus, Dyorhopapillomavirus, Dyosigmapapillomavirus, Dyothetapapillomavirus, Dyoxipapillomavirus, Dyozetapapillomavirus, Epsilonpapillomavirus, Etapapillomavirus, Gammapapillomavirus, Iotapapillomavirus, Kappapapillomavirus, Lambdapapillomavirus, Mupapillomavirus, Nupapillomavirus, Omegapapillomavirus, Omikronpapillomavirus, Phipapillomavirus, Pipapillomavirus, Psipapillomavirus, Rhopapillomavirus, Sigmapapillomavirus, Taupapillomavirus, Thetapapillomavirus, Upsilonpapillomavirus, Xipapillomavirus and Zetapapillomavirus.

Papillomaviridae viruses can in particular be the Human papillomavirus (HPV), more particularly HPV16, HPV39, HPV67, HPV68a or HPV53.

Polyomaviridae viruses can in particular be selected from the group consisting of the JC virus (JCV) found in a patient (initials JC), with Hodgkin's lymphoma who suffered from progressive multifocal leukoencephalopathy (PML); the BK virus (BKV) isolated from urine of a kidney transplant patient (initials BK); Merkel cell polyomavirus (MCPyV) associated with Merkel cell carcinoma; and the Trichodysplasia spinulosa-associated polyomavirus (TSPyV).

Polyomaviridae viruses can in particular be selected from the group consisting of the BK virus (BKV) and the Merkel cell polyomavirus (MCPyV).

Members of the Adenoviridae family include members of the genus Mastadenovirus, as for example Mastadenovirus A, Mastadenovirus B, Mastadenovirus C, Mastadenovirus D, Mastadenovirus E, Mastadenovirus F and Mastadenovirus G, which includes human adenoviruses.

They in particular include human adenovirus (HAdV)-A, HAdV-B, HAdV-C, HAdV-D, HAdV-E, and HAdV-F.

Parvoviridae viruses can be selected from the group consisting of Parvovirus, Erythrovirus, Dependovirus, Amdovirus, Bocavirus, Densovirus, Iteravirus, Brevidensovirus and Pefudensovirus.

Parvoviridae viruses are preferably an Erythovirus, and can more particularly be the Human parvovirus B19.

Reoviridae viruses can be selected from the group consisting of Orthoreovirus, Orbivirus, Rotavirus, Coltivirus, Aquareovirus, Cypovirus, Fijivirus, Phytoreovirus, Oryzavirus, Idnoreovirus and Mycoreovirus. Preferably, Reoviridae viruses are selected from Orbivirus, more preferably Kemerovo virus and Orungo virus; and Rotavirus, more preferably from Human rotavirus A.

An Hepevirus of the invention is in particular the Hepatitis E virus.

A Norovirus of the invention can in particular be selected from the group consisting of Human Norovirus saitama and human Norovirus.

An Enterovirus of the invention can in particular be selected from the group consisting of Coxsackievirus, in particular Coxsackievirus A20 and A16 ; Echo virus ; Enterovirus A71 ; Human rhinovirus; Rhinovirus C and Poliovirus, in particular human poliovirus 2.

A non-enveloped DNA virus of the invention can more preferably be selected from the group consisting of Mastadenovirus A; Mastadenovirus B; Mastadenovirus C; Mastadenovirus D; Mastadenovirus E; Mastadenovirus F; Mastadenovirus G; adenovirus; Human papillomavirus (HPV), in particular HPV16, HPV39, HPV67, HPV68a and HPV53; the BK virus (BKV); the Merkel cell polyomavirus (MCPyV); the Human parvovirus B19; Human rotavirus A; Kemerovo virus; Orungo virus; Hepatitis E; Human Norovirus saitama; human Norovirus; human poliovirus 2; Coxsackievirus A20; Coxsackievirus A16; Rhinovirus C; Human rhinovirus and Enterovirus A71.

A non-enveloped DNA virus of the invention is more preferably an adenovirus, in particular a human adenovirus.

Enveloped RNA viruses can in particular be selected from the group consisting of Filoviridae viruses, Bunyaviridae viruses, Flaviviridae viruses, Paramyxoviridae viruses, Poxviridae viruses, Alphatorquevirus viruses, Anneloviridae viruses, Coronaviridae viruses, Rubella virus, Alphavirus, Paramyxoviridae viruses, Rhabdoviridae viruses, Influenza A virus, Arenavirus, Bunyaviridae viruses, Retroviridae viruses and Hepadnaviridae viruses.

Filoviridae viruses, also called filoviruses, of the invention are in particular selected from the group consisting of Marburg virus, Ebola virus and Ravn virus.

Flaviviridae viruses of the invention are preferably selected from the group consisting of Hepacivirus, Flavivirus, Pegivirus and Pestivirus. They are more preferably selected from Hepacivirus, in particular Hepatitis C virus; and Flavivirus, in particular Dengue virus 4 and Dengue virus 5.

Poxviridae viruses of the invention can in particular be selected from the group consisting of Parapoxvirus, Betaentomopoxvirus, Yatapoxvirus, Cervidpoxvirus, Gammaentomopoxvirus, Leporipoxvirus, Suipoxvirus, Molluscipoxvirus, Crocodylidpoxvirus, Alphaentomopoxvirus, Capripoxvirus, Orthopoxvirus and Avipoxvirus.

Poxviridae viruses of the invention can preferably be selected from the group consisting of an Orthopoxvirus, in particular Cowpox virus, Vaccinia virus, Monkeypoxvirus and Variola virus; and a Parapoxvirus, in particular Orf virus.

Herpesviridae viruses of the invention can in particular be selected from the group consisting of Iltovirus, Proboscivirus, Cytomegalovirus, Mardivirus, Rhadinovirus, Macavirus, Roseolovirus, Simplexvirus, Scutavirus, Varicellovirus, Percavirus, Lymphocryptovirus and Muromegalovirus.

Herpesviridae viruses of the invention can preferably be selected from Varicellovirus, more preferably Human Herpesvirus 3; Simplexvirus, more preferably Human Herpesvirus 1; Cytomegalovirus, more preferably Human Herpesvirus 5; Roseolovirus, more preferably Human Herpesvirus 6A and Human Herpesvirus 7; and Lymphocryptovirus, more preferably Human Herpesvirus 4.

Annelloviridae viruses of the invention are in particular Alphatorquevirus, preferably Torque teno virus.

Coronaviridae viruses of the invention are in particular Coronavirinae viruses or Torovirinae viruses, preferably Coronavirus, in particular selected from the group consisting of SARS coronavirus sf098, human coronavirus OC43 and transmissible gastroenteritis virus.

Alphavirus of the invention can in particular be selected from the group consisting of Barmah Forest virus, Chikungunya virus, Mayaro virus, O'nyong'nyong virus, Ross River virus, Semliki Forest virus, Sindbis virus, Una virus, Eastern equine encephalitis virus, Tonate virus, Venezuelan equine encephalitis virus and Western equine encephalitis virus.

Alphavirus of the invention is preferably selected from Chikungunya virus and Ross River virus.

Paramyxoviridae viruses of the invention are preferably selected from the group consisting of Avulavirus, Morbillivirus, Aquaparamyxovirus, Henipavirus, Respirovirus, Rubulavirus, Ferlavirus, Pneumovirus and Metapneumovirus.

Paramyxoviridae viruses are preferably selected from the group consisting of Metapneumovirus, in particular human metapneumovirus; Pneumovirus, in particular Human respiratory syncytial virus; Rubulavirus, in particular Mumps virus; and Morbillivirus, in particular Measles virus.

Rhabdoviridae viruses are preferably selected from the group consisting of Lyssavirus, Novirhabdovirus, Ephemerovirus, Perhabdovirus, Tibrovirus, Nucleorhabdovirus, Tupavirus, Vesiculovirus, Sprivivirus, Cytorhabdovirus and Sigma virus.

Rhabdoviridae viruses are more particularly selected from the group consisting of Lyssavirus, in particular Vesicular stomatitis virus, Piry virus, Isfahan virus and Chandipura virus; and Vesiculovirus, in particular Mokola virus, Rabies virus, Aravan virus and Irkut virus.

Arenavirus of the invention can in particular be selected from the group consisting of Junin virus, Lymphocytic choriomeningitis virus, Lassa virus, Luna virus, Lunk virus, Dandenong virus, Mobala virus and Ippy virus.

Bunyaviridae viruses of the invention can in particular be selected from the group consisting of Batai virus; Hantavirus, in particular Dobrava-Belgrade virus; Nairovirus; Orthobunyavirus; Phlebovirus; and Tospovirus.

Retroviridae viruses of the invention can in particular be selected from the group consisiting of Alpharetrovirus, Betaretrovirus, Deltaretrovirus, Epsilonretrovirus, Gammaretrovirus, Lentivirus and Spumavirus.

Retroviridae viruses of the invention are more preferably selected from the group consisting of Lentivirus, in particular human immunodeficiency virus 1 and human immunodeficiency virus 2; Deltaretrovirus, in particular Human T-lymphotropic virus 1 (HTLV-1) and Human T-lymphotropic virus 2 (HTLV-2); and Alpharetrovirus, in particular Rous sarcoma virus and Rous associated virus.

Hepadnaviridae viruses of the invention are in particular selected from the group consisting of Avihepadnavirus and Orthohepadnavirus, and is more preferably Hepatitis B virus.

An enveloped RNA virus considered in the present invention is preferably selected from the group consisting of Orf virus, Cowpox virus, Vaccinia virus, Monkeypoxvirus, Variola virus, human herpesvirus 3, human herpesvirus 1, human herpesvirus 5, human herpesvirus 6A, human herpesvirus 7, human herpesvirus 4, Torque teno virus, Dengue virus 4, Dengue virus 5, Hepatitis C virus, SARS coronavirus sf098, Human coronavirus OC43, Transmissible gastroenteritis virus, Ross river virus, Chikungunya virus, Rubella virus, Ebola virus, Marburg virus, Ravn virus, Human metapneumovirus, human respiratory syncytial virus, Mumps virus, Measles virus, Vesicular stomatitis virus, Piry virus, Isfahan virus, Chandipura virus, Mokola virus, Rabies virus, Aravan virus, Irkut virus, Influenza A virus, Junin virus, Lymphocytic choriomeningitis virus, Lassa virus, Luna virus, Lunk virus, Dandenong virus, Mobala virus, Ippy virus, Batai virus, Dobrava-belgrade virus, HIV-1, HIV-2, HTLV-1, HTLV-2, Rous sarcoma virus, Rous associated virus and Hepatitis B virus.

An enveloped RNA virus considered in the present invention is more preferably a filovirus.

More particularly, a virus considered in the present invention can be selected in the group consisting of:

- a non-enveloped DNA virus of the invention can more preferably be selected from the group consisting of Mastadenovirus A; Mastadenovirus B; Mastadenovirus C; Mastadenovirus D; Mastadenovirus E; Mastadenovirus F; Mastadenovirus G; adenovirus; Human papillomavirus (HPV), in particular HPV16, HPV39, HPV67, HPV68a and HPV53; the BK virus (BKV); the Merkel cell polyomavirus (MCPyV); the Human parvovirus B19; Human rotavirus A; Kemerovo virus; Orungo virus; Hepatitis E; Human Norovirus saitama; human Norovirus; human poliovirus 2; Coxsackievirus A20; Coxsackievirus A16; Rhinovirus C; Human rhinovirus and Enterovirus A71; and
- an enveloped RNA virus considered in the present invention is preferably selected from the group consisting of Orf virus, Cowpox virus, Vaccinia virus, Monkeypoxvirus, Variola virus, human herpesvirus 3, human herpesvirus 1, human herpesvirus 5, human herpesvirus 6A, human herpesvirus 7, human herpesvirus 4, Torque teno virus, Dengue virus 4, Dengue virus 5, Hepatitis C virus, SARS coronavirus sf098, Human coronavirus OC43, Transmissible gastroenteritis virus, Ross river virus, Chikungunya virus, Rubella virus, Ebola virus, Marburg virus, Ravn virus, Human metapneumovirus, human respiratory syncytial virus, Mumps virus, Measles virus, Vesicular stomatitis virus,

Piry virus, Isfahan virus, Chandipura virus, Mokola virus, Rabies virus, Aravan virus, Irkut virus, Influenza A virus, Junin virus, Lymphocytic choriomeningitis virus, Lassa virus, Luna virus, Lunk virus, Dandenong virus, Mobala virus, Ippy virus, Batai virus, Dobrava-belgrade virus, HIV-1, HIV-2, HTLV-1, HTLV-2, Rous sarcoma virus, Rous associated virus and Hepatitis B virus.

A virus considered in the present invention is more preferably an adenovirus and/or a filovirus.

In a preferred embodiment, the compounds of the present invention can be used in a pharmaceutically acceptable carrier of a composition.

An object of the present invention is thus a composition comprising, in a pharmaceutically acceptable carrier, at least one compound of formula (II).

The routes of administration and dosage vary depending on a variety of parameters, for example depending on the individual's condition, the type of infection and the severity of infection to be treated or of compound(s) of the invention and of the other antiviral agents used. The compound according to the invention and the composition according to the invention are especially capable of being administered to an individual in dry, solid (in particular cachet, powder, capsule, pill, granule, suppository, or tablet polymer capsule, specifically accelerated release tablet, enteric tablet or prolonged-release tablets), under gelatinous form, or as a solution, or a liquid suspension (in particular syrup, injectable solution, or oral infusible, microvesicles, liposomes).

These compounds can also be in the form of doses in dry form (powder, lyophilizate, etc.) for reconstitution at the time of use using an appropriate diluents know to the man skilled in the art.

Depending on their dosage form, a composition of the invention may be administered enterally, parenterally (intravenously, intramuscularly or subcutaneous), transdermally (or percutaneous or transdermal), cutaneously, orally, mucosally, in particular transmucosally, buccally, nasally, ophthalmically, otologically (in the ear), esophageally, vaginally, rectally, or intragastrically, intracardiacally, intraperitoneally, intrapulmonaryly or intratracheally.

Furthermore, the compound of the invention or the composition of the invention may be packaged for administration as a single dose (single dose) or multiple (multidose).

To enhance the effects of treatment, it is possible to proceed with an administration in the form of several successive administrations, repeated at one or more occasions after a particular time interval. One can, for example, carry out several administrations per day or per week.

The amount of active ingredient administered to an individual is in a therapeutically effective amount.

A "therapeutically effective amount" is an amount sufficient to produce a significant effect, especially bring a significant benefit to said individual as part of an administration for the prophylaxis or treatment as defined previously.

A therapeutically effective amount is an amount for which the beneficial effects outweigh any toxic or detrimental effect of or ingredient(s) active(s). The therapeutically effective amount will vary depending on factors such as the state of infection, age, sex or weight of the individual.

Dosage regimens may be adjusted to obtain an optimum therapeutic effect.

More specifically, a therapeutically effective amount of compound of structure I of the invention may be between 40 mg/day and 1600 mg/day, in particular between 80 mg/day and 1200 mg/day, more particularly between 100 mg/day and 800 mg/day, preferably between 200 mg/day and 500 mg/day, administered for example in 1 to 3 doses.

The present invention is illustrated by the following examples, given purely for illustrative purposes, with reference to the following figures.

**Example 1: Preparation of CT2-10 (1-((2,2",4,4"-Tetrachloro-[1,1':3',1"-terphenyl]-4'-yl)methyl)-1H-imidazole)**

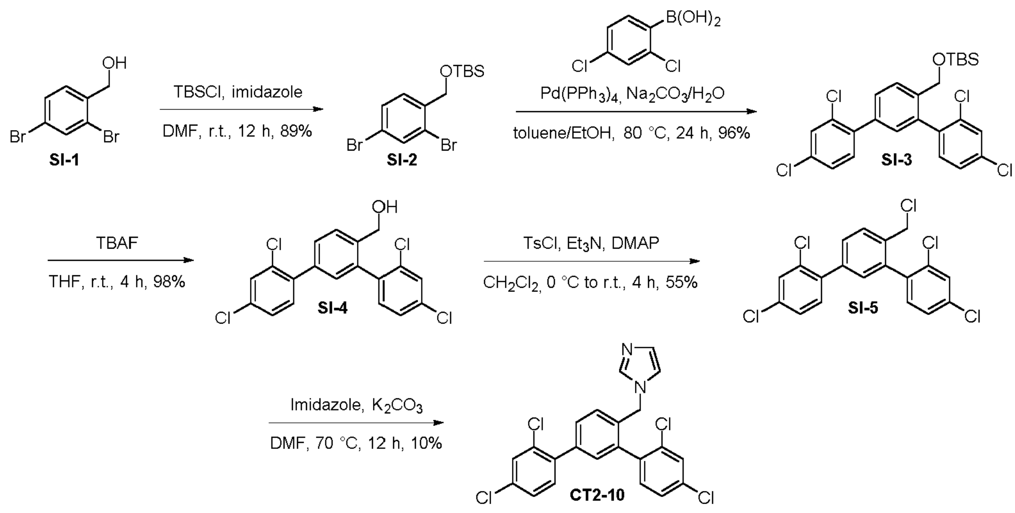


**tert-Butyl((2,4-dibromobenzyl)oxy)dimethylsilane (SI-2)**

To a stirred solution of commercially available 2,4-dibromobenzyl alcohol SI-1 (500 mg, 1.88 mmol) in DMF (4 mL), under inert atmosphere, were added imidazole (153 mg, 2.25 mmol, 1.2 eq.) and TBSCl (340 mg, 2.25 mmol, 1.2 eq.). The resulting mixture was stirred for 12 h, then hydro lyzed with water and extracted with a mixture 9:1 cyclohexane/$CH_2Cl_2$ (three times). The combined organic extracts were washed with water, brine, dried over $MgSO_4$, filtered and concentrated under reduced pressure. The crude product was then purified by chromatography (100% petroleum ether) to afford the title compound SI-2 (638 mg, 1.68 mmol, 89%) as a colorless oil.

$^1$H NMR ($CDCl_3$, 300 MHz): δ 7.66 (d, J = 1.8 Hz, 1H), 7.49-7.42 (m, 2H), 4.67 (s, 2H), 0.97 (s, 9H), 0.14 (s, 6H).

**tert-Butyldimethyl((2,2",4,4"-tetrachloro-[1,1':3',1"-terphenyl]-4'-yl)methoxy)silane (SI-3)**

A two-neck flask equipped with a stirring bar and a condenser was charged with previously described SI-2 (600 mg, 1.58 mmol) and toluene (16 mL). After bubbling argon in the reaction mixture for 5 min, $Pd(PPh_3)_4$ (55 mg, 47 μmol, 3 mol%) was added and the system was capped. An aqueous solution of $Na_2CO_3$ (3.15 mL, 2 M, 6.31 mmol, 4 eq.) was then added, followed by slow addition of a solution of 2,4-dichlorobenzeneboronic acid (603 mg, 3.15 mmol, 2 eq.) in absolute EtOH (12.5 mL). The resulting mixture was heated to 80 °C and stirred at this temperature for 12 h, time after which the reaction mixture was charged again with $Pd(PPh_3)_4$ catalyst (55 mg, 47 μmol, 3 mol%) and stirred for additional 12 h at 80 °C. The reaction slurry was then diluted with water and extracted with $CH_2Cl_2$ (five times). The combined organic extracts were dried over $MgSO_4$, filtered and concentrated under reduced pressure. The crude product was purified by flash chromatography (0% to 5% $Et_2O$ in petroleum ether) to give the desired coupled product SI-3 (778 mg, 1.52 mmol, 96%) as a colorless oil.

$^1$H NMR ($CDCl_3$, 300 MHz): δ 7.69 (d, J = 8.1 Hz, 1H), 7.51-7.47 (3H), 7.34-7.30 (3H), 7.24 (d, J = 8.1 Hz, 1H), 7.18 (d, J = 2.0 Hz, 1H), 4.57 (d, J = 13.5 Hz, 1H), 4.45 (d, J = 13.5 Hz, 1H), 0.91 (s, 9H), 0.03 (s, 3H), 0.02 (s, 3H).

$^{13}$C NMR ($CDCl_3$, 75 MHz): δ 138.9, 138.4, 137.5, 136.7, 135.9, 134.1 (2×C), 133.7, 133.2, 132.1, 132.0, 130.4, 129.8, 129.3, 129.2, 127.2, 127.0, 126.8, 62.5, 25.9 (3×C), 18.3, -5.4, -5.5.

**(2,2",4,4"-Tetrachloro-[1,1':3',1"-terphenyl]-4'-yl)methanol (SI-4):**

To a stirred solution of previously described SI-3 (750 mg, 1.46 mmol) in THF (5 mL), under inert atmosphere, was added TBAF (2.19 mL, 1 M in THF, 2.19 mmol, 1.5 eq.). The reaction mixture was stirred at r.t. for 4 h, time after which a saturated aqueous NH4Cl solution was added. The reaction mixture then diluted with $Et_2O$ and extracted with this solvent (three times). The combined organic extracts were washed with brine, dried over $MgSO_4$, filtered, and concentrated under reduced pressure. The residue was purified by flash chromatography (0% to 10% EtOAc in petroleum ether) to afford the title benzylic alcohol SI-4 (570 mg, 1.43 mmol, 98%) as a colorless oil.

$^1$H NMR ($CDCl_3$, 300 MHz): δ 7.68 (d, J = 8.1 Hz, 1H), 7.54-7.49 (3H), 7.35-7.30 (3H), 7.27 (d, J = 8.1 Hz, 1H), 7.23 (d, J = 1.8 Hz, 1H), 4.58 (d, J = 13.0 Hz, 1H), 4.47 (d, J = 13.0 Hz, 1H).

$^{13}$C NMR ($CDCl_3$, 75 MHz): δ 138.4, 138.1, 137.5, 137.3, 136.8, 134.3, 134.1, 133.9, 133.2, 132.1, 132.0, 130.8, 129.8, 129.6, 129.4, 127.8, 127.2, 127.1, 62.7.

**2,2",4,4"-Tetrachloro-4'-(chloromethyl)-1,1':3',1"-terphenyl (SI-5)**

To a stirred solution of previously described SI-4 (100 mg, 0.25 mmol) in dry $CH_2Cl_2$ (1 mL), under inert atmosphere, were added DMAP (6.1 mg, 51.0 μmol, 0.2 eq.) and $Et_3N$ (47 μL, 0.35 mmol, 1.4 eq.). The reaction mixture was cooled down to 0 °C and TsCl (67 mg, 0.35 mmol, 1.4 eq.) was added. The resulting mixture was stirred at r.t. for 4 h, then hydrolyzed with water, diluted with $CH_2Cl_2$ and extracted with this solvent (three times). The combined organic extracts were dried over $MgSO_4$, filtered and concentrated under reduced pressure. The crude product was purified by flash chromatography (0% to 10% EtOAc in petroleum ether) to afford SI-5 (57 mg, 0.14 mmol, 55%) as a colorless oil

$^1H$ NMR ($CDCl_3$, 300 MHz): δ 7.64 (d, J = 7.9 Hz, 1H), 7.55-7.48 (3H), 7.37-7.34 (2H), 7.33-7.31 (2H), 7.26 (d, J = 1.8 Hz, 1H), 4.53 (d, J = 11.7 Hz, 1H), 4.34 (d, J = 11.7 Hz, 1H).

**1-((2,2",4,4"-Tetrachloro-[1,1':3',1"-terphenyl]-4'-yl)methyl)-1H-imidazole (CT2-10)**

To a stirred solution of previously described SI-5 (53 mg, 0.13 mmol) in DMF (0.5 mL), under inert atmosphere, were added $K_2CO_3$ (26 mg, 0.19 mmol, 1.5 eq.) and imidazole (13 mg, 0.19 mmol, 1.5 eq.). The reaction mixture was heated to 70 °C and stirred for 12 h. Water was then added to the reaction mixture which was extracted with $CH_2Cl_2$ (three times). The combined organic extracts were washed with brine, dried over $MgSO_4$, filtered and concentrated under reduced pressure. The crude product was purified by preparative TLC (petroleum ether/EtOAc/$Et_3N$ 60:38:2) to afford the desired product CT2-10 as a colorless oil.

$^1H$ NMR (acetone-$d_6$, 300 MHz): δ 7.66 (d, J = 2.0 Hz, 1H), 7.62 (m, 1H), 7.56 (dd, J = 8.0, 2.0 Hz, 1H), 7.52-7.45 (3H), 7.41 (br s, 1H), 7.37 (d, J = 8.0 Hz, 1H), 7.35-7.31 (2H), 6.95-6.90 (2H), 5.18 (d, J = 15.5 Hz, 1H), 5.10 (d, J = 15.5 Hz, 1H).

$^{13}C$ NMR (acetone-$d_6$, 75 MHz):i δ 139.1, 138.8, 138.4, 138.1, 136.3, 135.2, 134.9, 134.7, 133.9, 133.6, 133.5, 132.0, 130.7, 130.5, 130.2, 129.8, 129.3, 128.6, 128.5, 48.7.

**Example 2: Preparation of CT2-13 (1-(2,5-bis(2,4-Dichlorophenyl)furan-3-yl)-1H-imidazole)**

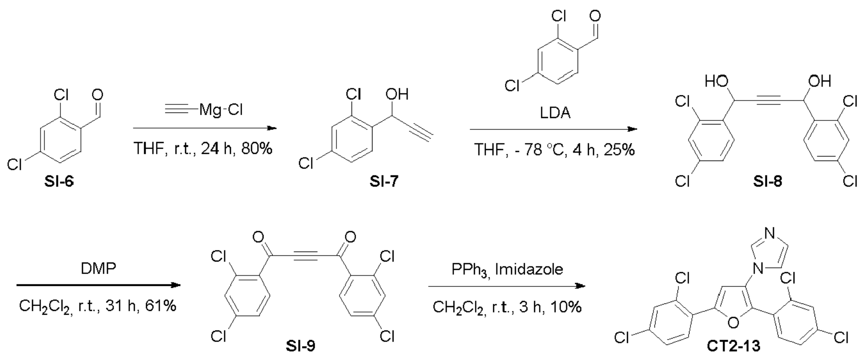


**1-(2,4-Dichlorophenyl)prop-2-yn-1-ol (SI-7)**

To a stirred solution of commercially available 2,4-dichlorobenzaldehyde (2.5 g, 14.3 mmol) in freshly distilled THF (27 mL), under inert atmosphere, was added dropwise ethynylmagnesium chloride (34.3 mL, 0.5 M in THF, 17.1 mmol, 1.2 eq.). The resulting mixture was stirred at r.t. for 24 h, then quenched with a saturated aqueous $NH_4Cl$ solution, diluted with EtOAc and extracted with solvent (three times). The combined organic extracts were washed with water, brine, dried over $MgSO_4$, filtered and concentrated under reduced pressure. The residue was purified by flash chromatography (0% to 10% EtOAc in petroleum ether) to give the title propargylic alcohol SI-7 (2.3 g, 11.4 mmol, 80%) as a pale yellow oil.

$^1H$ NMR ($CDCl_3$, 300 MHz): δ 7.73 (d, J = 8.3 Hz, 1H), 7.42 (d, J = 2.0 Hz, 1H), 7.32 (dd, J = 8.3, 2.0 Hz, 1H), 5.78 (dd, J = 5.3, 2.3 Hz, 1H), 2.67 (d, J = 2.3 Hz, 1H), 2.48 (d, J = 5.3 Hz, 1H).

**1,4-bis(2,4-Dichlorophenyl)but-2-yne-1,4-diol (SI-8)**

To a stirred solution of freshly prepared LDA (6.2 mL, 1 M in THF, 6.2 mmol, 2.5 eq.), under inert atmosphere, was added dropwise a solution of previously described SI-7 (500 mg, 2.49 mmol) in anhydrous THF (2.5 mL) at - 78 °C.

The reaction mixture was stirred at - 78 °C for 1 h, then a solution of 2,4-dichlorobenzaldehyde (522 mg, 2.98 mmol, 1.2 eq.) in anhydrous THF (3 mL) was added dropwise. The resulting mixture was stirred at - 78 °C for additional 2 h, time after which it was quenched with a saturated aqueous $NH_4Cl$ solution, diluted with EtOAc and extracted with this solvent (three times). The combined organic extracts were washed with water (twice), brine, dried over $MgSO_4$, filtered and concentrated under reduced pressure. The residue was purified by flash chromatography (0% to 30% EtOAc in petroleum ether) to afford the desired diol SI-8 (230 mg, 0.61 mmol, 25%) as a white powder.

$^1H$ NMR (DMSO-$d_6$, 300 MHz): δ 7.68 (dd, J = 8.4, 2.0 Hz, 2H), 7.60 (m, 2H), 7.48 (dd, J = 8.4, 2.0 Hz, 2H), 6.34 (m, 2H), 5.58 (m, 2H).

$^{13}C$ NMR (DMSO-$d_6$, 75 MHz): δ 138.2 (2×C), 133.1 (2×C), 132.2 (2×C), 129.4 (2×C), 128.7 (2×C), 127.5 (2×C), 84.4 (2×C), 59.4 (2×C).

**1,4-bis(2,4-Dichlorophenyl)but-2-yne-1,4-dione (SI-9)**

To a stirred solution of previously described SI-8 (100 mg, 0.27 mmol) in $CH_2Cl_2$ was added Dess-Martin periodinane (338 mg, 0.80 mmol, 3 eq.). The reaction mixture was stirred at r.t. for 12 h, time after which portions of Dess-Martin periodinane (56 mg, 0.13 mmol, 0.5 eq.) were added every 3-4 h until reaching a complete conversion (followed by TLC, petroleum ether/EtOAc 8:2, 2-3 additions). A 1:1 mixture of saturated aqueous $NH_4Cl$ and saturated $Na_2S_2O_3$ solutions was then added and the reaction slurry was stirred at r.t. for additional 15 min. The organic layer was separated, the aqueous layer was then extracted with $CH_2Cl_2$ (three times) and the combined organic extracts were washed with brine, dried over $MgSO_4$, filtered and concentrated under reduced pressure. The crude product was purified by flash chromatography (petroleum ether/EtOAc 9:1) to give the title diketone SI-9 (59.4 mg, 0.16 mmol, 61%) as a white solid.

$^1H$ NMR ($CDCl_3$, 300 MHz): δ 8.04 (d, J = 8.5 Hz, 2H), 7.55 (d, J = 2.0 Hz, 2H), 7.43 (dd, J = 8.5, 2.0 Hz, 2H).

$^{13}C$ NMR ($CDCl_3$, 75 MHz): δ 173.6 (2×C), 140.8 (2×C), 135.3 (2×C), 134.1 (2×C), 132.2 (2×C), 131.8 (2×C), 127.6 (2×C), 87.0 (2×C).

**1-(2,5-bis(2,4-Dichlorophenyl)furan-3-yl)-1H-imidazole (CT2-13)**

To a stirred solution of $PPh_3$ (35.2 mg, 0.13 mmol, 1 eq.) and imidazole (9.2 mg, 0.13 mmol, 1 eq.) in dry $CH_2Cl_2$ (0.3 mL), under inert atmosphere, was added dropwise a solution of previously described SI-9 (50 mg, 0.13 mmol) in dry $CH_2Cl_2$ over 15 min. The reaction mixture was stirred at r.t. for 3 h then concentrated under reduced pressure. The residue was purified by preparative TLC (petroleum ether/EtOAc 1:1) to isolate a first fraction which was purified again by preparative TLC ($CH_2Cl_2/Et_2O$ 9:1) to afford the desired furan CT2-13 (5.6 mg, 13.2 μmol, 10%) as a pale yellow solid.

$^1H$ NMR (acetone-$d_6$, 300 MHz): δ 8.07 (d, J = 6.6 Hz, 1H), 8.05 (s, 1H), 7.69 (d, J = 8.4 Hz, 1H), 7.66 (d, J = 2.0 Hz, 1H), 7.63-7.61 (2H), 7.56 (dd, J = 8.5, 2.0 Hz, 1H), 7.53 (s, 1H), 7.52 (dd, J = 8.5, 2.0 Hz, 1H), 7.17 (m, 1H).

$^{13}C$ NMR (acetone-$d_6$, 75 MHz): δ 146.7, 140.3, 138.7, 137.4, 135.6, 134.7, 133.5, 133.4, 132.5, 132.4, 132.1, 131.1, 128.6, 126.1, 118.6, 115.6, 106.0.

**Example 3: Inhibition of adenoviral propagation by Sulconazole**

Adenoviral propagation was quantified by fluorescent plaque forming assay with GFP expressing HAd-C5 vector in the presence of the compounds as a measure of virus spread through multiple rounds of infection.

a) Method

HEK293 cells were infected with 0.1 physical particles per cell (pp/cell) of E1-deficient GFP expressing HAd-C5-GFP vector for 24 hours followed by removal of the inoculums and overlay with 1% agarose supplemented with vehicle only (DMSO) or 5 μM of Sulconazole.

Six days after infection, plaque sizes were measured by epifluorescence microscopy using Image J.

b) Statistics

Statistical analysis was carried out using unpaired Student t-test.

c) Results

The results obtained are presented in Figure 1.

The analysis showed significant reduced plaque size, and thus a significant reduction of the adenovirus virus

propagation, when the assay was carried out in the presence of Sulconazole compared to a negative control only supplemented with DMSO.

**Example 4: Inhibition of VP40-mediated filovirus egress by Sulconazole**

a) Method

HEK293 cells were transfected with 1μg pC-VP40 constructs: MARV VP40 containing the wild type (wt) VP40 of Marburg virus ($VP40_{WT}$) or MARV VP40 with the late domain motif of Ebola virus (PTAPPEY) VP40 ($VP40_{ELD}$), or an empty plasmid as negative control, using Lipofactamine 2000 (Invitrogen).

These cells were incubated with either DMSO or with 5μM of Sulconazole. N4 (5 μM) was used as positive control. This compound has indeed previously been identified as reducing the egress of VP40 VLPs (Han Z et al., Journal of Virology 88(13): 7294-7306).

24 hours later, VLP and cells lysates were prepared as described in Han Z et al. (Journal of Virology 88(13): 7294-7306), and analyzed by Western blotting using goat anti-VP40 antibody and mouse anti-GAPDH (Hitest).

Signal intensities were collected using a LAS (GE Healthcare) and quantified using Image J.

The cellular VP40 signal ($VP40_{cell}$) was normalized using the:

$$\text{GAPDH signal intensity} = \text{Normalized } VP40_{cell} = VP40_{cell} / GAPDH_{mean}.$$

Then, VLP release efficiency was calculated as:

$$\text{VLP release efficiency} = [(\text{VP40supernatant} - \text{VP40supernatant mock}) + (\text{Normalized } VP40_{cell} - \text{Normalized } VP40_{cell\ mock})]$$

b) Statistics

Statistical analysis was carried out using unpaired Student t-test.

c) Results

The analysis of VLP VP40 content by Western blot revealed that both N4 and Sulconazole inhibit both MARV $VP40_{WT}$ (Figure 2) and $VP40_{ELD}$ (Figure 3) derived VLP release, with Sulconazole presenting the highest efficiency in both cases.

**Example 5: Inhibition of the replication of infectious filovirus by Sulconazole**

a) Method

HUH-7 cells were infected at an MOI of 0.1 with MARV in presence of different concentrations (5 μM and 10 μM) of Sulconazole or the vehicle control or added the compounds following entry at 5 hours post-infection to the cells.

24 hours post-infection and 48 hours post-infection, viral supernatants were collected and titers were determined by TCID50 endpoint titration using the Spearman-Karber method.

b) Statistics

Statistical analysis was carried out using unpaired Student t-test.

c) Results

The results obtained are represented in Figure 3.

At 24 hours post-infection, Sulconazole strongly reduces MARV titers by >1-2log.

Inhibition via Sulconazole occurred irrespective of the time the drug was added.

**Example 6: Sulconazole inhibits adenovirus infection at an early stage**

The effect of Sulconazole was determined on the onset of adenoviral gene expression by quantifying the intracellular expression levels of the immediate early gene E1A and two early genes, E1B and E4 orf6/7.

a) Method

U2OS cells were infected for 4 hours at 37 °C with replication competent HAd-C5-wt virus in medium containing 5 μM of Sulconazole or a vehicle control.

4 hours post-infection, total RNA was extracted, reverse transcribed and early viral genes E1A, E1B and E4 orf6/7 gene expression was determined by real-time PCR using the primers indicated in Table 1 hereafter.

| Primer Name | *Sequence number* | *Sequence* |
|---|---|---|
| GAPDH fwd | *SEQ ID NO: 1* | *5'-TGGTATCGTGGAAGGACTCA-3'* |
| GAPDH rev | *SEQ ID NO: 2* | *5'-CCAGTAGAGGCAGGGATGAT-3'* |
| E1B-55k fwd | *SEQ ID NO: 3* | *5'-GAGGGTAACTCCAGGGTGCG-3'* |
| E1B-55k rev | *SEQ ID NO: 4* | *5'-TTTCACTAGCATGAAGCAACCACA-3'* |
| E4orf6/7 fwd | *SEQ ID NO: 5* | *5'-ACAGAACCCTAGTATTCAACCTGC-3'* |
| E4orf6/7 rev | *SEQ ID NO: 6* | *5'-GACAGCGACATGAACTTAAGTGAG-3'* |
| E1A fwd | *SEQ ID NO: 7* | *5'-GGCTCAGGTTCAGACACAGGACTGTAG-3'* |
| E1A rev | *SEQ ID NO: 8* | *5'-TCCGGAGCCGCCTCACCTTTC-3'* |

**Table 1. Real-time PCR primers used**

b) Statistics

Statistical analysis was carried out using unpaired Student t-test.

c) Results

The results obtained are indicated in Figure 4.

A significant reduction of early viral genes expression is revealed 4 hours post-infection when cells where treated with Sulconazole.

It thus appears clearly that Sulconazole specifically inhibits adenovirus infection at an early stage.

**Example 7: Sulconazole reduces the nuclear accumulation of adenoviral genomes**

The efficiency of viral genome nuclear import was quantified upon treatment with Sulconazole.

a) Method

U2OS cells grown on coverslips were pre-treated for 1 hour with 5 μM of Sulconazole or vehicle alone. Infections were done for 30 minutes at 37 °C with 2500 pp/cell HAd-C5 in medium containing 5 μM of Sulconazole or DMSO followed by inoculums removal and addition of fresh drug containing media.

2 hours post-infection, coverslips were fixed and nuclear protein VII was detected using specific antibodies prepared and characterized as indicated in Komatsu et al. PLoS One. 2015 Sep 2;10(9):e0137102 and Alexa488 conjugated secondary antibodies.

Cells were analyzed by confocal microscopy and the protein VII signal was quantified using an automated macro in Image J.

b) Statistics

Statistical analysis was carried out using unpaired Student t-test.

c) Results

The results obtained are indicated in Figure 5.
Quantification of protein VII decorating viral genomes 2 hours after-infection, when genome nuclear import reaches a plateau, revealed that Sulconazole significantly reduces the nuclear accumulation of viral genomes.

**Example 8: Inhibition of adenoviral propagation by Sulconazole and its derivatives Oxiconazole, CT2-10 and CT2-13 and comparison of their activity compared to a lead compound: Cidofovir™**

Sulconazole and three of its derivatives, Oxiconazole, CT2-10 and CT2-13, are tested for their anti-adenoviral activity using the plaque assay described in Example 1.
Cidofovir™, the standard clinical drug used against adenovirus infections in the clinic, is also included in this test.
The compounds were used at a concentration of 5 μM. Cidofovir was in particular used at even higher concentration, i.e. 10 and 20 μM.
DMSO was used as negative control.

a) Statistics

Statistical analysis was carried out using unpaired Student t-test.

b) Results

The results obtained are illustrated in Figure 6.
Compared to the DMSO negative control, Sulconazole and its derivatives demonstrated an effective anti-adenoviral activity.
Moreover, these compounds of the invention demonstrated an increased activity compared to the lead compound Cidofovir.
In contrast, Cidofovir did not show significant inhibition even at highest non-cytotoxic concentrations (10 μM and 20 μM).
Accordingly, the inventors identified a novel class of compounds having a strong anti-viral activity.
Moreover, Oxiconazole, CT2-10 and CT2-13 showed cytotoxicity profiles similar to Sulconazole (see Figure 7), which is not cytotoxic at a concentration of 5 μM on U2OS cells.
To determine said cytotoxicity, U2OS cells were treated for 36 h with 250 nM to 50 μM of each compound (Sulconazole, CT2-10, CT2-13 and Oxiconazole). Cell viability in the presence of the candidate drugs was monitored using the CellTiter-Glo® Luminescent Cell Viability Assay (Promega) according to the manufacturer's instructions. Cell viability was normalized to the mean of the signal obtained in presence of DMSO alone. The mean values were determined from a triplicate experiment and error bars are SD.

**Example 9: Sulconazole interacts with the NEDD4 peptide and the imidazole ring is essential for this interaction to occur**

Sulconazole is now shown herein to be active against adenovirus and filovirus.
On the other hand, it was previously reported that the NEDD4/[LP]PxY interaction contributes to efficient entry of non-enveloped adenoviruses (Wodrich et al. ; 2010; A Capsid-Encoded PPxY-Motif Facilitates Adenovirus Entry. PLoS Pathog. 6, e1000808). Also, NEDD4/[LP]PxY interaction has been exploited as drug targets for enveloped RNA viruses (Han et al. ; Small-Molecule Probes Targeting the Viral PPxY-Host Nedd4 Interface Block Egress of a Broad Range of RNA Viruses. J. Virol. 88, 7294-7306 ; 2014).
Accordingly, the inventors sought for the mechanism of action responsible for the antiviral activity of Sulconazole, and derivatives thereof, by investigating their ability to modulate the interaction between NEDD4 and peptides bearing a [LP]PxY motif.
To this end they have used solution nuclear magnetic resonance spectroscopy (NMR) to obtain structural

information. They have synthesized a peptide corresponding to the 45 amino acids of WW3-domain of NEDD4 and for which an NMR solution structure bound to a PPxY containing peptide from EBOV was previously obtained (PDB ID: 2KQ0). Subsequently NMR analysis was performed in absence and presence of Sulconazole.

a) Methods

**NMR.** The 1D and 2D 1H NMR experiments (Total Correlation Spectroscopy (TOCSY), Nuclear Overhauser Effect Spectroscopy (NOESY) Correlation Spectroscopy (COSY) and the and 2D 1H-13C heteronuclear NMR experiments Heteronuclear Single Quantum Coherence (HSQC) and Heteronuclear Single Quantum Coherence-Nuclear Overhauser Effect Spectroscopy (HSQC-NOESY) were performed at 850.13 MHz on a Bruker 850 MHz instrument equipped with an UltraShield Plus magnet and a triple resonance cryoprobe with gradient unit. The 2D NMR experiments were performed at 300 K without spinning with mixing times of 110 ms for the TOCSY experiments and 250 ms for the NOESY and HSQC-NOESY experiments, respectively.

**Docking studies.** The crystallographic coordinates of human NEDD4 third WW domain complexed with Ebola Zaire Virus Matix protein VP40 derived peptide (PDB id 2KQ0). The peptide ligand was removed and the protein was prepared using MOE 2015.10 [Molecular Operating Environment (MOE). 2015, Chemical Computing Group: Montreal, Canada.]. To this end, hydrogen atoms were added using MOE's protonate 3D facility with the dielectric constants set to 3 inside the protein and 80 for the solvent (water). The temperature was set to 310 K, the salt concentration to 0.2 M and the pH to 7.0. The Lennard-Jones 12-6 potential was used for simulating van der Waals interactions, whereas electrostatic interactions were simulated according to the GB/VI model (Gilson, M.K. (1993). Multiple-site titration and molecular modeling: two rapid methods for computing energies and forces for ionizable groups in proteins. Proteins 15, 266-282.; Labute, P. (2008). The generalized Born/volume integral implicit solvent model: estimation of the free energy of hydration using London dispersion instead of atomic surface area. J. Comput. Chem. 29, 1693-1698).

Cutoff distances were set to 15 Å and 10 Å for the electrostatic and van der Waals interactions, respectively. The structure was energy minimized using the AMBER12 force field (Case et al. 2012) to within an rms gradient of 0.01 kcal $mol^{-1}$ $Å^{-1}$. Throughout, all systems were surrounded by a distance dependent dielectric model of bulk water. The resulting protein model was used in the docking studies.

Three peptide ligands were obtained from the corresponding crystal structures, 2KQ0, 2LAJ, and 2MPT, whereas the two other peptides (DEPPSYEE and DEPGAAEE) were built in MOE and prepared following the described methodology. The four small molecules, Sulconazole, Oxiconazole, CT2-13 and CT2-10 were built in MOE and energy minimized using the AMBER12 forcefield to within an rms gradient of 0.001 kcal $mol^{-1}$ $Å^{-1}$. The peptide ligands were docked into the human NEDD4 protein, employing the protein-protein docking module in MOE, selecting a pocket in NEDD4 as receptor (the cavity formed by two antiparallel β-sheets) and the different peptides as ligands. The protein-protein docking facility generates different poses using FFTs (Fast Fourier Transforms) followed by all atom minimization. Finally, the small ligands were computationally docked in the same pocket of NEDD4 using the alpha triangle placement methodology with affinity $\Delta G$ as scoring function as implemented in MOE. In the docking studies with small molecules, flexible ligand structures were generated using a Monte Carlo algorithm, whereas the receptor was held fixed according to the minimized geometry.

**Molecular Dynamics simulations.** All molecular dynamics simulations of the peptide - NEDD4 complexes were performed using the YASARA Structure program (Krieger, 2004-2016, YASARA). The ligand-model complexes as obtained from the docking studies were energy minimized to within an rms gradient of 0.1 kcal $mol^{-1}$ $Å^{-1}$ using the Amber ff03 molecular mechanics force field (Duan et al. (2003). A point-charge force field for molecular mechanics simulations of proteins based on condensed-phase quantum mechanical calculations. J. Comput. Chem. 24, 1999-2012).

The models were then solvated and minimized in a box of water extending 10 Å from the nearest atoms of the complex, giving approximately 30 000 atoms in each simulation box. Taking pKa values into account, protonation states were assigned at physiologic pH 7.4 (Krieger et al. (2012). Assignment of protonation states in proteins and ligands: combining pKa prediction with hydrogen bonding network optimization. Methods Mol. Biol. Clifton NJ 819, 405-421).

In order to achieve physiological salt concentration, water molecules were randomly replaced by sodium or chloride ions and the solvent density adjusted to 0.997 g $L^{-1}$, followed by Molecular Dynamics (MD) simulation and final energy minimization step. The MD simulation was performed for 50 ns using periodic boundary conditions and the NVT canonical ensemble at 298 K. The simulation was carried out using the multiple time steps 2.5 fs for intermolecular forces and 1.25 fs for intramolecular ones. The particle mesh Ewald (PME) method was used for long-range electrostatics with a cutoff of 7.86 Å (Linse and Linse (2014) Tuning the smooth particle mesh Ewald sum: application on ionic solutions and dipolar fluids. J. Chem. Phys. 141, 184114). Trajectory snapshots were sampled every 100 ps during the MD simulation.

b) Results

The NMR analyses showed that Sulconazole interacts with the NEDD4 peptide and that the imidazole ring is essential for this interaction to occur. When equimolar amounts of Sulconazole was added to the NEDD4 peptide the chemical shift values of the imidazole ring of Sulconazole changed significantly when compared with that of pure Sulconazole dissolved in the same solvent at the same concentrations, whereas the chemical shift values of the remaining aromatic hydrogens and carbon atoms mainly remained unchanged. The NMR data suggested binding between Sulconazole and the NEDD4 peptide involved the imidazole ring and amino acids W39 and F28.

Exploiting this information, we performed a docking study with an active site bias derived from the NMR data on the same structure model (2KQ0) we used for the peptide/protein interaction studies. Sulconazole efficiently docked on the exposed [LP]PxY peptide binding surface of the WW-domain with the imidazole ring inserting into a cavity formed by W39 and F28 thus confirming the NMR data and providing a structural rational for the interaction.

Importantly, when the docking predictions for the viral DEPPSYEE peptide and Sulconazole were superimposed on the NEDD4 structure the imidazole ring occupied the same cavity that was occupied by the essential second Proline of the [LP]PxY motif while one of the Sulconazole aromatic rings occupied the same binding interface as the essential Tyrosine of the motif (Figure 6 A). Molecular docking analysis of the other imidazole derivatives using the same docking bias for the W39/F28 cavity as for Sulconazole revealed a similar mode of binding for Oxiconazole, while structural constrain of the two other compounds CT2-10 and CT2-13 did not allow complete insertion into the cavity. However, superimposition of the predicted docking sites for the 4 imidazole derivatives suggested that all compounds interfere with the substrate binding.

In conclusion, NMR spectroscopy analysis using a synthetic peptide encoding the WW3 domain of NEDD4 showed direct interaction between the Imidazole ring of Sulconazole and a binding pocket on the exposed substrate binding surface of WW3. Most importantly *in silico* docking analysis suggested that Sulconazole (and its derivatives) occupied an essential binding sites for the [LP]PxY motif, thus acting as allosteric competitor for the binding of [LP]PxY motif containing substrates.

The NMR based structure model also provides evidence that imidazole-derivatives with higher affinity and specificity can be generated via targeted rational drug design ; which thus validates the broad antiviral effect of compound of formula (I).

## Claims

**1.** A compound of formula (I):

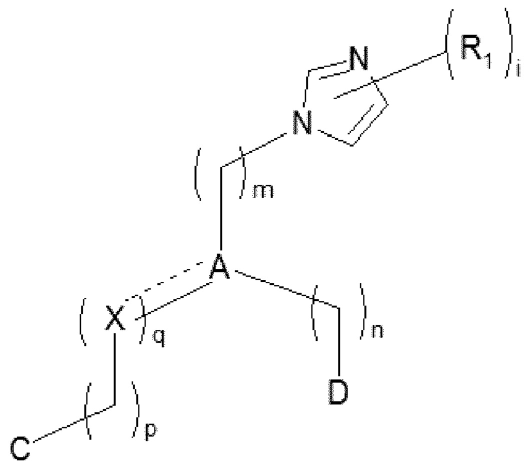

Formula (I)

wherein:

---- represents a single bond or a double bond;
q is 0 or 1;
A represents:

- CH when (i) ---- represents a single bond with q being 1 or (ii) when q is 0;
- a carbon atom when ---- represents a double bond and q is 1; or
- a heteroaromatic ring or an aromatic ring, when (i) ---- represents a single bond with q being 1, or when (ii) q is 0 ;

X, being present when q is 1, represents:

-S-;
-O-;

a -O-NH- group with ---- representing a single bond, A being linked either to the nitrogen atom or to the oxygen atom of the -O-NH- group; or
a -O-N- group with ---- representing a double bond, A being linked to the nitrogen atom of the -O-N- group;
C and D, independently, represent a non-substituted aromatic ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p, independently, are 0, 1 or 2;
i is 1, 2 or 3; and
$R_1$ independently represents a hydrogen atom, a halogen atom or a $C_1$-$C_4$ alkyl group;

or one of its pharmaceutically acceptable salts;
for use in the prevention and/or treatment of viral infections by a non-enveloped DNA virus and/or an enveloped RNA virus,
said compound of formula (I) being in all the possible racemic, enantiomeric and diastereoisomeric isomer forms.

**2.** The compound for use according to claim 1, wherein A represents a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl.

**3.** The compound for use according to claim 1 or 2, wherein A is selected from a furyl group and a phenyl group.

**4.** The compound for use according to anyone of the preceding claims, wherein X represents:

- S-; or

a -O-N- group with ---- representing a double bond, A being linked to the nitrogen atom of the -O-N- group.

5. The compound for use according to anyone of the preceding claims, wherein C and D, independently, represent a phenyl ring, in particular a phenyl ring substituted with one or two halogen atom, preferably one or two chlorine atom(s).

6. The compound for use according to anyone of the preceding claims, wherein m is 1, n is 0, p is 1 and q is 1.

7. The compound for use according to anyone of claims 1 to 5, wherein m is 0 or 1 and n, p and q are 0.

8. The compound for use according to claim 1, wherein the compound is selected from the group consisting of:

- Sulconazole or one of its pharmaceutically acceptable salts;
- Oxiconazole or one of its pharmaceutically acceptable salts; and
- a compound of formula (II):

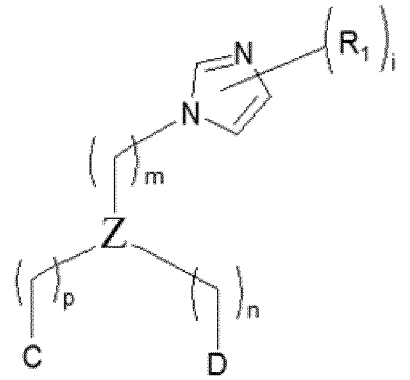

Formula (II)

wherein C, D, m, n, p, i and $R_1$ are as defined in the compound of Formula (I) in claim 1; and
Z represents a saturated or unsaturated 5- or 6-membered ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl;

or one of its pharmaceutically acceptable salts;
said compound of formula (II) being in all the possible racemic, enantiomeric and diastereoisomeric isomer forms.

9. The compound for use according to anyone of the preceding claims, wherein the compound is selected from the group consisting of:

- Sulconazole or one of its pharmaceutically acceptable salts;
- Oxiconazole or one of its pharmaceutically acceptable salts; and
- a compound of formula (I) wherein:
- C and D are both a phenyl ring substituted with two chlorine groups;
- m is 0 or 1;
- i is 1;
- n, p and q are 0;
- $R_1$ represents an hydrogen atom; and
- A represents a furyl or a phenyl group;

or one of its pharmaceutically acceptable salts,
said compound being in all the possible racemic, enantiomeric and diastereoisomeric isomer forms.

10. The compound for use according to anyone of the preceding claims, wherein it is for use in the prevention and/or

treatment of viral infections by an adenovirus and/or a filovirus.

**11.** A compound of formula (I):

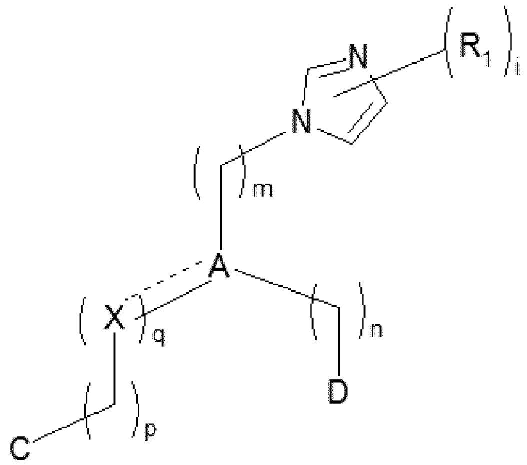

wherein:

---- represents a single bond or a double bond;
q is 0 or 1;
A represents:

- CH when (i) ---- represents a single bond with q being 1 or (ii) when q is 0;
- a carbon atom when ---- represents a double bond and q is 1; or
- a heteroaromatic ring or an aromatic ring, when (i) ---- represents a single bond with q being 1, or when (ii) q is 0 ;

X, being present when q is 1, represents:

-S-;
-O-;

a -O-NH- group with ---- representing a single bond, A being linked either to the nitrogen atom or to the oxygen atom of the -O-NH- group; or
a -O-N- group with ---- representing a double bond, A being linked to the nitrogen atom of the -O-N- group;
C and D, independently, represent a non-substituted aromatic ring or an aromatic ring substituted by one or more substituents independently selected from the group consisting of a halogen atom, a $C_1$-$C_4$ alkyl group, a $C_1$-$C_4$ alkoxy group, a hydroxyl group, a -$NO_2$ group, a -$NR_2R_3$ group and a -CN group, with $R_2$ and $R_3$ being independently selected from a hydrogen atom and a $C_1$-$C_4$ alkyl group;
m, n and p, independently, are 0, 1 or 2;
i is 1, 2 or 3; and
$R_1$ independently represents a hydrogen atom, a halogen atom or a $C_1$-$C_4$ alkyl group;

or one of its pharmaceutically acceptable salts,
for use in the prevention and/or treatment of viral infections,
said compound of formula (I) being in all the possible racemic, enantiomeric and diastereoisomeric isomer forms;
with the proviso that said compound of formula (I) is not of formula:

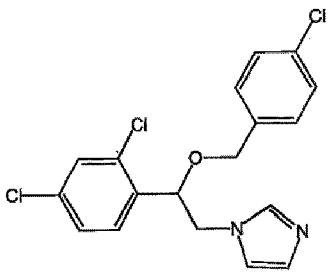
.

**12.** The compound for use according to anyone of the preceding claims, wherein it is used in a pharmaceutically acceptable carrier of a composition.

**13.** A compound of formula (II):

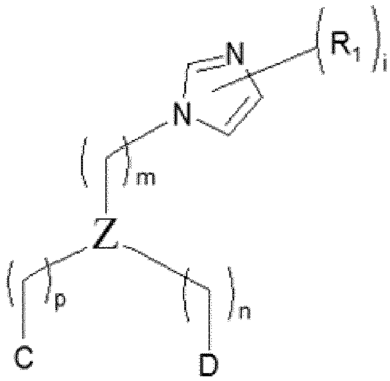

Formula (II)

wherein :

C and D, independently, represent a non-substituted ring or an aromatic ring substituted by one or more substituents independently selected from the group consisting of a halogen atom, a $C_1$-$C_4$ alkyl group, a $C_1$-$C_4$ alkoxy group, a hydroxyl group, a $-NO_2$ group, a $-NR_2R_3$ group and a -CN group, with $R_2$ and $R_3$ being independently selected from a hydrogen atom and a $C_1$-$C_4$ alkyl group;
m, n and p independently are 0, 1 or 2;
i is 1, 2 or 3;
$R_1$ represents, independently, a hydrogen atom, a halogen atom or a $C_1$-$C_4$ alkyl group, preferably a hydrogen atom; and
Z represents a saturated or unsaturated 5- or 6-membered ring, in particular a ring selected from the group consisting of phenyl, imidazolyl, pyrazolyl, thiazolyl, oxazolyl, isothiazolyl, isoxazolyl, furyl, thiophenyl, oxadiazolyl, thiadiazolyl, triazolyl, tetrazolyl, pyridinyl, pyrazinyl, pyrimidinyl, pyridazinyl and triazinyl group;

or one of its pharmaceutically acceptable salts,
said compound of formula (II) being in all the possible racemic, enantiomeric and diastereoisomeric isomer forms.

**14.** The compound according to claim 13, wherein C, D, m, n and p are as defined in anyone of claims 2 to 7.

**15.** The compound according to claim 13 or 14, selected from the group consisting of:

(i) a compound wherein:

- C and D both represent a phenyl ring substituted with two chlorine groups;
- m, n and p are 0;
- Z represents a furyl group; and

- $R_1$ represents an hydrogen atom and i is 1;

and
(ii) a compound wherein:

- C and D both represent a phenyl ring substituted with two chlorine groups;
- m is 1;
- n and p are 0;
- Z is a phenyl group; and
- $R_1$ represents an hydrogen atom and i is 1.

**16.** A composition comprising, in a pharmaceutically acceptable carrier, at least one compound of formula (II) as defined in anyone of claims 13 to 15.

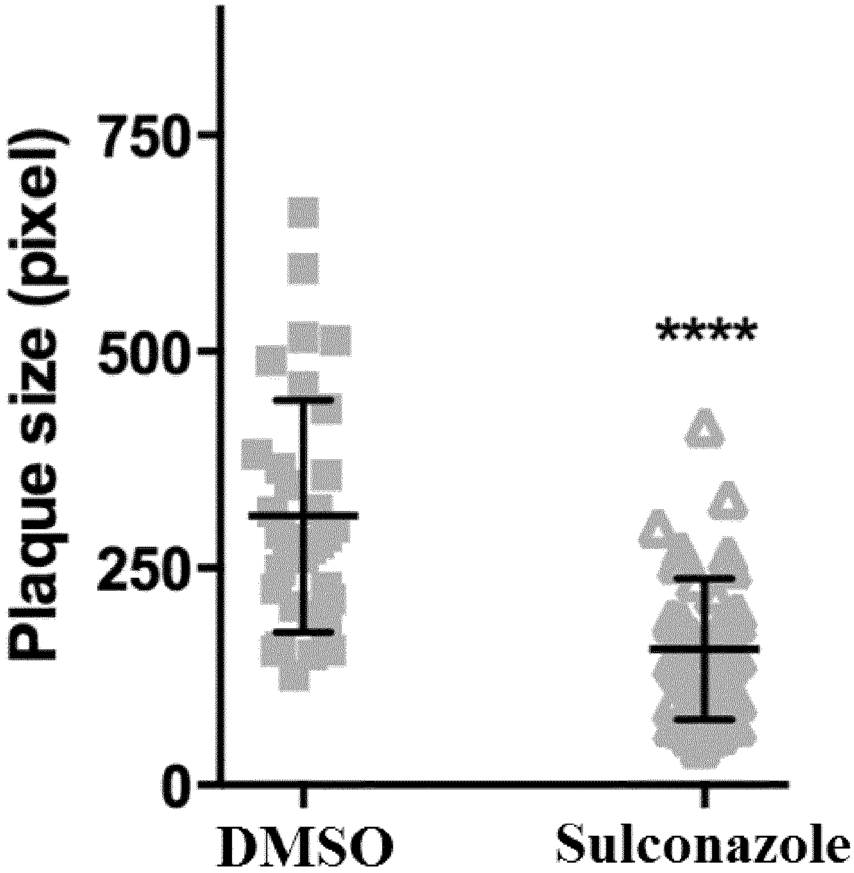


FIGURE 1

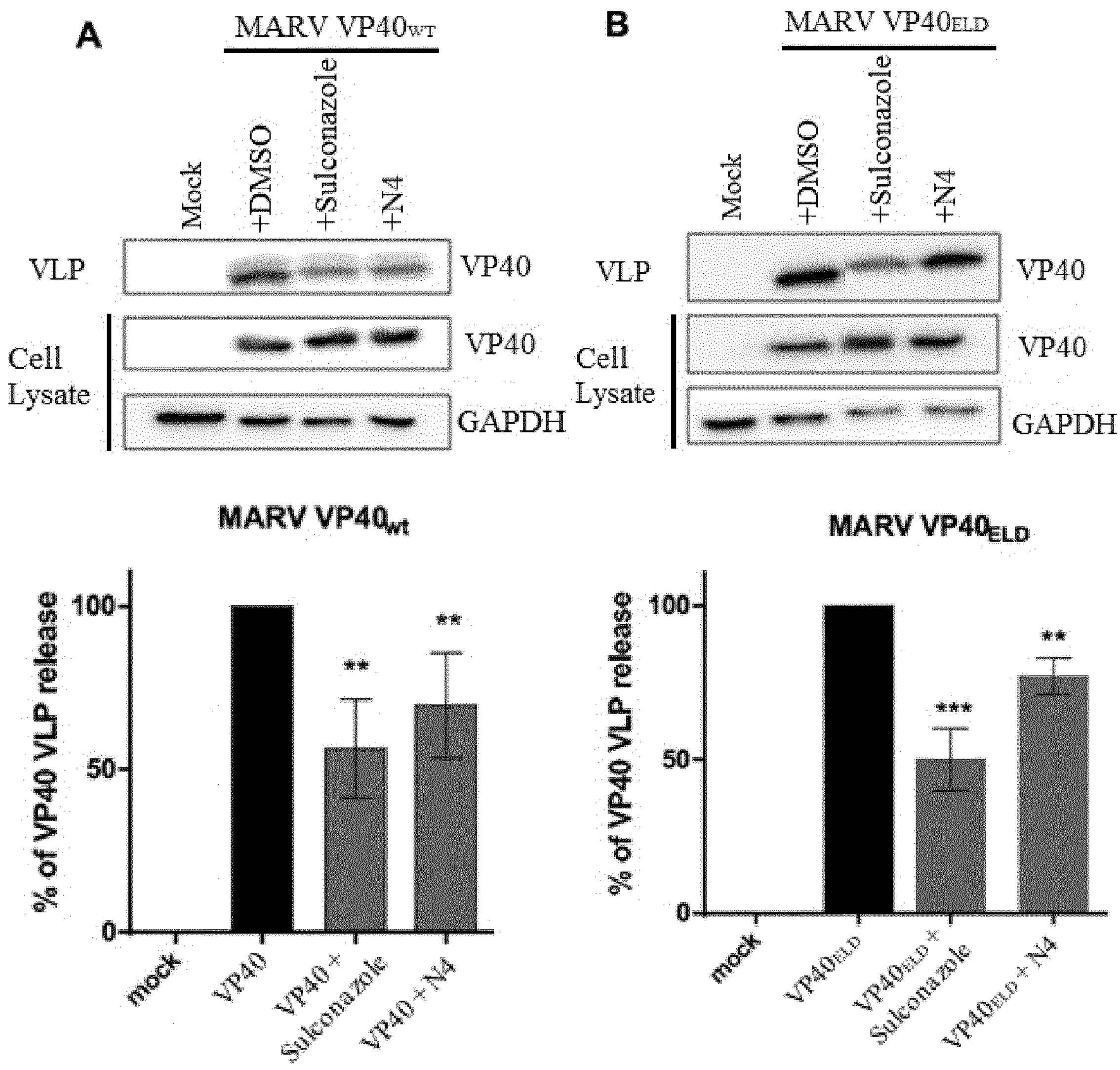
A
MARV VP40WT
Mock
+DMSO
+Sulconazole
+N4
VLP
VP40
Cell
Lysate
VP40
GAPDH
B
MARV VP40ELD
Mock
+DMSO
+Sulconazole
+N4
VLP
VP40
Cell
Lysate
VP40
GAPDH
MARV VP40wt
% of VP40 VLP release
100
50
0
**
**
mock
VP40
VP40 + Sulconazole
VP40 + N4
MARV VP40ELD
% of VP40 VLP release
100
50
0
***
**
mock
VP40ELD
VP40ELD + Sulconazole
VP40ELD + N4

**FIGURE 2**

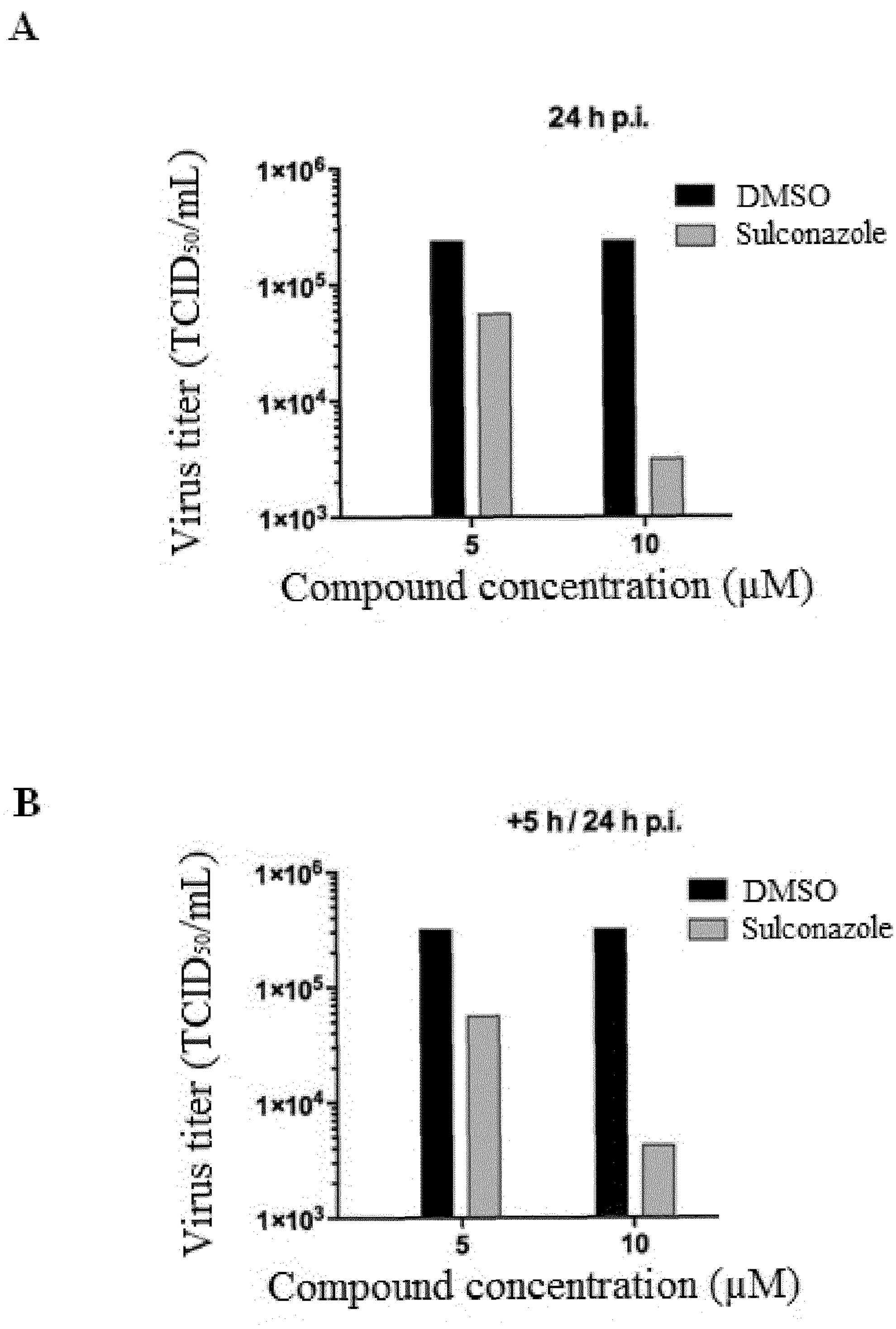
A
24 h p.i.
Virus titer (TCID50/mL)
1×10^6
1×10^5
1×10^4
1×10^3
DMSO
Sulconazole
5
10
Compound concentration (μM)
B
+5 h / 24 h p.i.
Virus titer (TCID50/mL)
1×10^6
1×10^5
1×10^4
1×10^3
DMSO
Sulconazole
5
10
Compound concentration (μM)

FIGURE 3

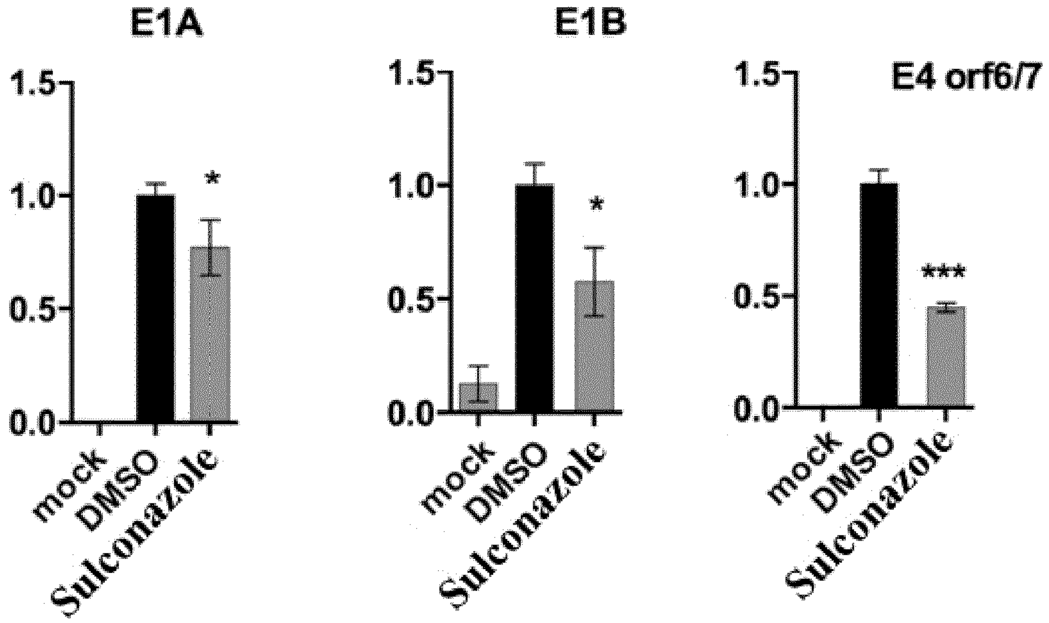
E1A
1.5
1.0
0.5
0.0
*
mock
DMSO
Sulconazole
E1B
1.5
1.0
0.5
0.0
*
mock
DMSO
Sulconazole
E4 orf6/7
1.5
1.0
0.5
0.0
***
mock
DMSO
Sulconazole

FIGURE 4

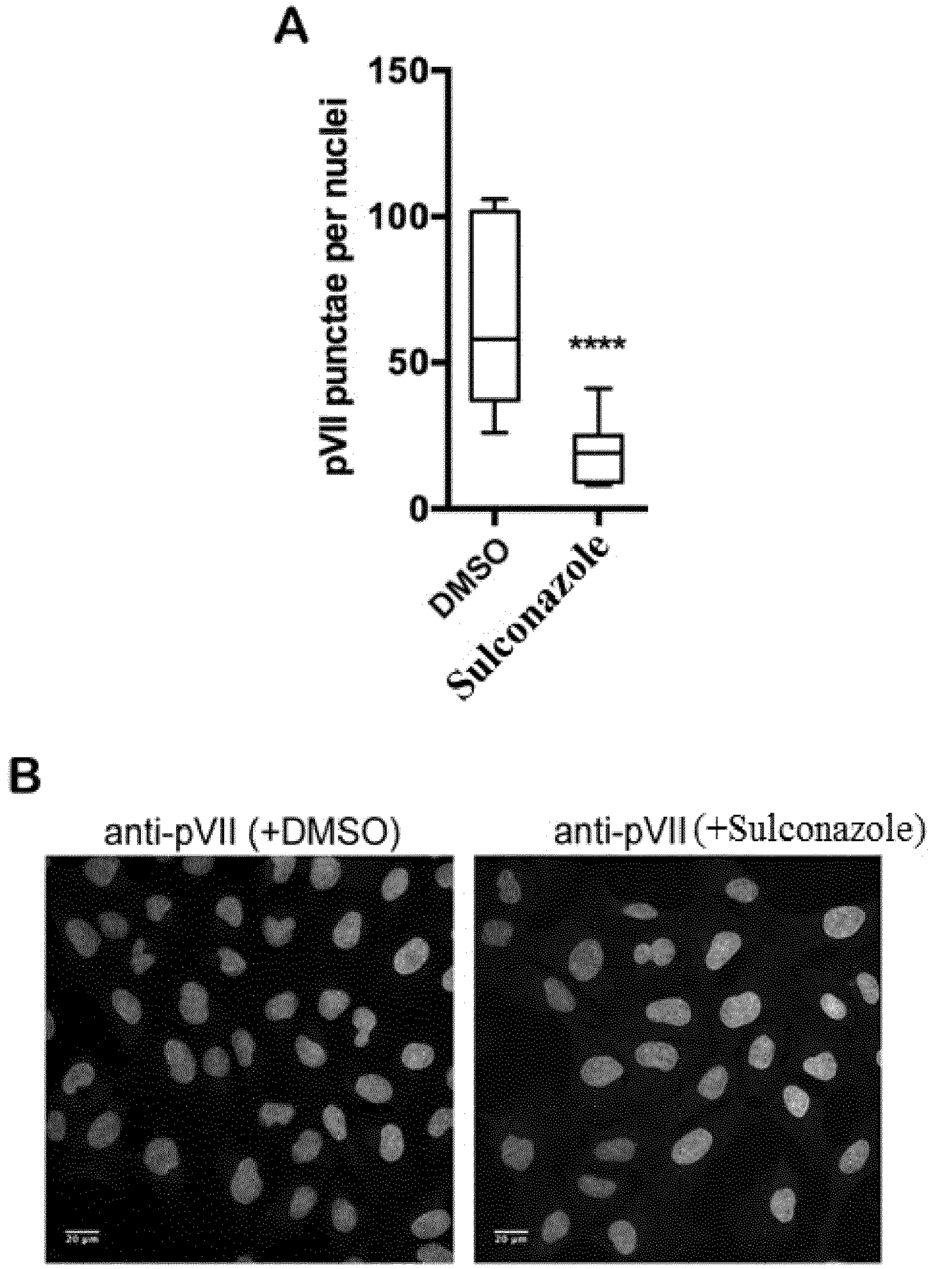
A
150
100
50
0
pVII punctae per nuclei
****
DMSO
Sulconazole
B
anti-pVII (+DMSO)
anti-pVII (+Sulconazole)

FIGURE 5

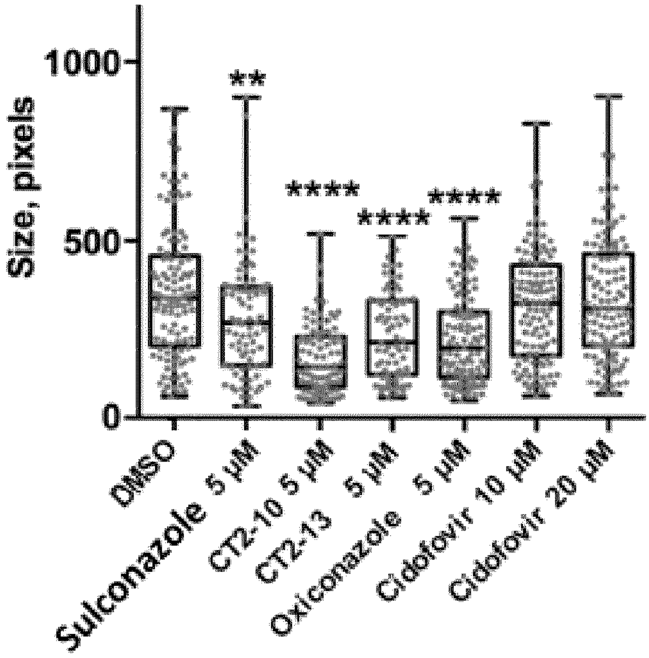
Size, pixels
1000
500
0
**
****
****
****
DMSO
Sulconazole 5 μM
CT2-10 5 μM
CT2-13 5 μM
Oxiconazole 5 μM
Cidofovir 10 μM
Cidofovir 20 μM

FIGURE 6

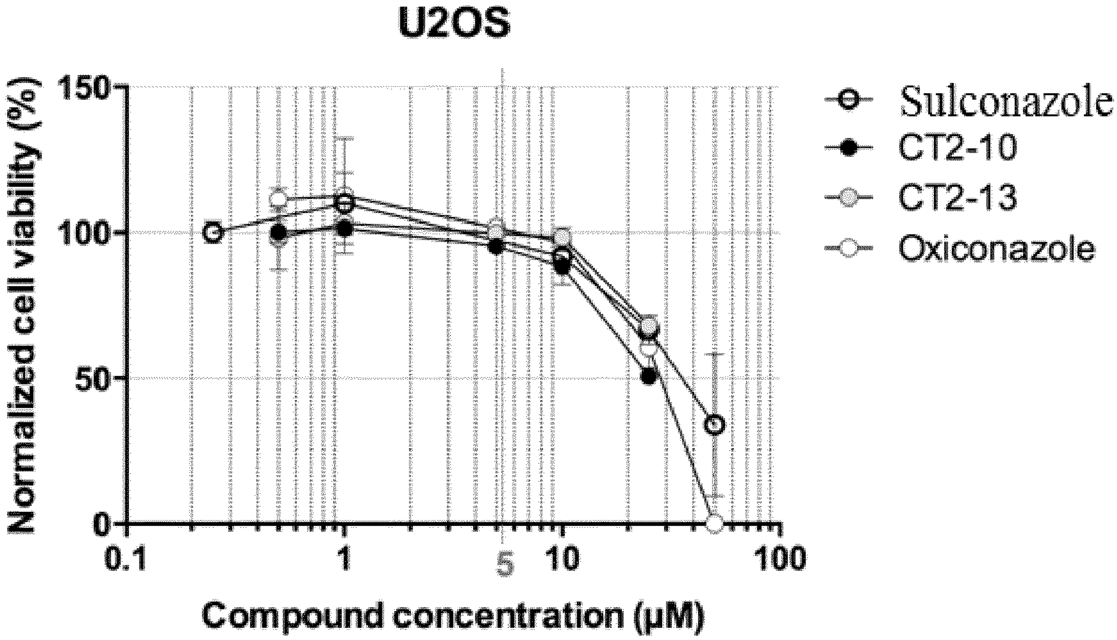
U2OS
Normalized cell viability (%)
150
100
50
0
0.1
1
5
10
100
Compound concentration (µM)
Sulconazole
CT2-10
CT2-13
Oxiconazole

FIGURE 7

Europäisches Patentamt
European Patent Office
Office européen des brevets

# EUROPEAN SEARCH REPORT

Application Number
EP 17 30 5371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 03/063869 A1 (MACFARLANE BURNET INST FOR MED [AU]; ANDERSON DAVID ANDREW [AU]; GAZIN) 7 August 2003 (2003-08-07) * pages 1-3 - pages 17-19; compound VI * ----- | 1-16 | INV. C07D233/56 C07D233/60 C07D405/04 A61K31/4164 A61K31/4178 A61P31/12 |
| Y | US 2007/219239 A1 (MJALLI ADNAN M [US] ET AL) 20 September 2007 (2007-09-20) * pages 1-2; examples * ----- | 1-16 | |
| Y | US 5 910 506 A (SUGIMOTO HIROHIKO [JP] ET AL) 8 June 1999 (1999-06-08) * pages 1-2; examples * ----- | 1-16 | |
| A | US 2011/028564 A1 (JOHANSEN LISA M [US] ET AL) 3 February 2011 (2011-02-03) * pages 1-2; examples * ----- | 1-16 | |
| A | KUJAWSKI JACEK ET AL: "Structural and spectroscopic properties of econazole and sulconazole - Experimental and theoretical studies", JOURNAL OF MOLECULAR STRUCTURE, ELSEVIER, AMSTERDAM, NL, vol. 1119, 26 April 2016 (2016-04-26), pages 250-258, XP029564807, ISSN: 0022-2860, DOI: 10.1016/J.MOLSTRUC.2016.04.065 * pages 250-251; figure 1 * ----- | 1-16 | TECHNICAL FIELDS SEARCHED (IPC): C07D |
| | -/-- | | |

The present search report has been drawn up for all claims

1

| Place of search | Date of completion of the search | Examiner |
|---|---|---|
| Munich | 17 July 2017 | Lauro, Paola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 30 5371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BHANDARI K ET AL: "Tetrahydronaphthyl azole oxime ethers: The conformationally rigid analogues of oxiconazole as antibacterials", EUROPEAN JOURNAL OF MEDICINAL CHEMISTRY, EDITIONS SCIENTIFIQUE ELSEVIER, PARIS, FR, vol. 44, no. 1, 1 January 2009 (2009-01-01), pages 437-447, XP025842186, ISSN: 0223-5234, DOI: 10.1016/J.EJMECH.2008.01.006 [retrieved on 2008-01-25] * figure 1; table 1 * ----- | 1-16 | |
| A | EP 0 666 077 A1 (MEIJI SEIKA CO [JP]; BIOMATERIAL CO LTD [JP]) 9 August 1995 (1995-08-09) * claim 1 * ----- | 1-16 | |
| X | YAVARI I ET AL: "Reaction between heterocyclic NH-acids and dibenzoylacetylene in the presence of triphenylphosphine. Simple synthesis of 1-(3-furyl)-1H-imidazole derivatives", TETRAHEDRON LET, ELSEVIER, AMSTERDAM, NL, vol. 43, no. 51, 16 December 2002 (2002-12-16), pages 9449-9452, XP004392997, ISSN: 0040-4039, DOI: 10.1016/S0040-4039(02)02259-1 * compound 3a * ----- -/-- | 13 | TECHNICAL FIELDS SEARCHED (IPC) |

The present search report has been drawn up for all claims

1

| Place of search | Date of completion of the search | Examiner |
|---|---|---|
| Munich | 17 July 2017 | Lauro, Paola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 30 5371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU J ET AL: "A modified procedure for the synthesis of 1-arylimidazoles", SYNTHESIS, GEORG THIEME VERLAG, STUTTGART, DE, no. 17, 1 January 2003 (2003-01-01), pages 2661-2666, XP002429212, ISSN: 0039-7881, DOI: 10.1055/S-2003-42444 * compound 15 * ----- | 13 | |
| X | ULRIKE E HILLE ET AL: "First selective CYP11B1 Inhibitors for the Treatment of Cortisol-Dependent Diseases", ACS MEDICINAL CHEMISTRY LETTERS, AMERICAN CHEMICAL SOCIETY, UNITED STATES, vol. 2, no. 1, 13 January 2011 (2011-01-13), pages 2-6, XP002635800, ISSN: 1948-5875, DOI: 10.1021/ML100071J [retrieved on 2010-10-22] * compound 12 * ----- | 13 | |
| | | | TECHNICAL FIELDS SEARCHED (IPC) |

1 The present search report has been drawn up for all claims

| Place of search | Date of completion of the search | Examiner |
|---|---|---|
| Munich | 17 July 2017 | Lauro, Paola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 17 30 5371

This annex lists the patent family members relating to the patent documents cited in the above-mentioned European search report. The members are as contained in the European Patent Office EDP file on
The European Patent Office is in no way liable for these particulars which are merely given for the purpose of information.

17-07-2017

| Patent document cited in search report | Publication date | Patent family member(s) | Publication date |
|---|---|---|---|
| WO 03063869 A1 | 07-08-2003 | CA 2474821 A1 | 07-08-2003 |
| | | CN 1638772 A | 13-07-2005 |
| | | EP 1480648 A1 | 01-12-2004 |
| | | JP 2005522425 A | 28-07-2005 |
| | | NZ 534292 A | 26-09-2008 |
| | | US 2005080122 A1 | 14-04-2005 |
| | | WO 03063869 A1 | 07-08-2003 |
| US 2007219239 A1 | 20-09-2007 | US 2007219239 A1 | 20-09-2007 |
| | | WO 2008054454 A2 | 08-05-2008 |
| US 5910506 A | 08-06-1999 | AT 255564 T | 15-12-2003 |
| | | AU 706095 B2 | 10-06-1999 |
| | | BR 9509024 A | 30-09-1997 |
| | | CA 2200316 A1 | 04-04-1996 |
| | | CN 1158609 A | 03-09-1997 |
| | | DE 69532245 D1 | 15-01-2004 |
| | | DE 69532245 T2 | 16-09-2004 |
| | | DK 0786455 T3 | 29-03-2004 |
| | | EP 0786455 A1 | 30-07-1997 |
| | | ES 2211917 T3 | 16-07-2004 |
| | | FI 971234 A | 23-05-1997 |
| | | JP 3155009 B2 | 09-04-2001 |
| | | KR 100387157 B1 | 29-09-2003 |
| | | NO 971306 A | 21-05-1997 |
| | | PL 320009 A1 | 01-09-1997 |
| | | PT 786455 E | 27-02-2004 |
| | | RU 2157368 C2 | 10-10-2000 |
| | | TW 401404 B | 11-08-2000 |
| | | US 5910506 A | 08-06-1999 |
| | | US 6147097 A | 14-11-2000 |
| | | WO 9610019 A1 | 04-04-1996 |
| US 2011028564 A1 | 03-02-2011 | US 2011028564 A1 | 03-02-2011 |
| | | US 2013289024 A1 | 31-10-2013 |
| EP 0666077 A1 | 09-08-1995 | EP 0666077 A1 | 09-08-1995 |
| | | JP 2875140 B2 | 24-03-1999 |
| | | JP H0753372 A | 28-02-1995 |
| | | WO 9505171 A1 | 23-02-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03063869 A


### Non-patent literature cited in the description


- **E. W. MARTIN.** Remington's Pharmaceutical Sciences. Mack Publishing Co, 1990
- **K.L. KIMMEL et al.** *J. Am. Chem. Soc.,* 2009, vol. 131, 8754
- **G. BARTOLI et al.** *Tetrahedron Lett.,* 1994, vol. 35, 8651
- **M. JEAN et al.** *Org. Biomol. Chem.,* 2015, vol. 13, 9168-9175
- **MATSUOKA et al.** *Tetrahedron,* 2006, vol. 62, 8199-8206
- **ALTMAN et al.** *J. Org. Chem.,* 2007, vol. 72, 6190-6199
- **HAN Z et al.** *Journal of Virology,* vol. 88 (13), 7294-7306
- **KOMATSU et al.** *PLoS One,* 02 September 2015, vol. 10 (9), e0137102
- **WODRICH et al.** A Capsid-Encoded PPxY-Motif Facilitates Adenovirus Entry. *PLoS Pathog.,* 2010, vol. 6, e1000808
- **HAN et al.** Small-Molecule Probes Targeting the Viral PPxY-Host Nedd4 Interface Block Egress of a Broad Range of RNA Viruses. *J. Virol.,* 2014, vol. 88, 7294-7306
- Molecular Operating Environment (MOE). Chemical Computing Group, 2015
- **GILSON, M.K.** Multiple-site titration and molecular modeling: two rapid methods for computing energies and forces for ionizable groups in proteins. *Proteins,* 1993, vol. 15, 266-282
- **LABUTE, P.** The generalized Born/volume integral implicit solvent model: estimation of the free energy of hydration using London dispersion instead of atomic surface area. *J. Comput. Chem.,* 2008, vol. 29, 1693-1698
- **DUAN et al.** A point-charge force field for molecular mechanics simulations of proteins based on condensed-phase quantum mechanical calculations. *J. Comput. Chem.,* 2003, vol. 24, 1999-2012
- **KRIEGER et al.** Assignment of protonation states in proteins and ligands: combining pKa prediction with hydrogen bonding network optimization. *Methods Mol. Biol. Clifton NJ,* 2012, vol. 819, 405-421
- **LINSE ; LINSE.** Tuning the smooth particle mesh Ewald sum: application on ionic solutions and dipolar fluids. *J. Chem. Phys.,* 2014, vol. 141, 184114